# EUROPÄISCHE PATENTSCHRIFT

(11) **EP 0 625 975 B1**
(45) Veröffentlichungstag und Bekanntmachung des Hinweises auf die Patenterteilung: **14.08.1996**
(21) Anmeldenummer: 93917360.5
(22) Anmeldetag: 30.01.1993
(51) Int. Cl.: C07D 309/06, C07D 335/02, C07C 251/54, C07C 323/47, A01N 43/16, A01N 43/18, A01N 35/10

(54) **CYCLOHEXENONOXIMETHER, VERFAHREN ZU IHRER HERSTELLUNG UND IHRE VERWENDUNG ALS HERBIZIDE**
CYCLOHEXENONE OXIME ETHERS, PROCESS FOR MAKING THEM AND THEIR USE AS HERBICIDES
ETHERS DE CYCLOHEXENONE-OXIMES, LEUR PROCEDE DE PREPARATION ET LEUR UTILISATION COMME HERBICIDES

(30) Priorität: 13.02.1992 DE 4204205
(43) Veröffentlichungstag der Anmeldung: 30.11.1994
(73) Patentinhaber: BASF Aktiengesellschaft, 67063 Ludwigshafen (DE)
(72) Erfinder: MISSLITZ, Ulf, D-6730 Neustadt (DE); MEYER, Norbert, D-6802 Ladenburg (DE); KAST, Juergen, D-6737 Boehl (DE); BOTT, Kaspar, D-6800 Mannheim 1 (DE); WALTER, Helmut, D-6719 Obrigheim (DE); WESTPHALEN, Karl-Otto, D-6720 Speyer (DE); KARDORFF, Uwe, D-6800 Mannheim 1 (DE); GERBER, Matthias, D-6704 Mutterstadt (DE)
(86) Internationale Anmeldenummer: EP9300215
(87) Internationale Veröffentlichungsnummer: WO9316063

(56) Entgegenhaltungen:
- EP-A- 0 456 112

## Beschreibung

Die vorliegende Erfindung betrifft neue Cyclohexenonoximether der allgemeinen Formel I 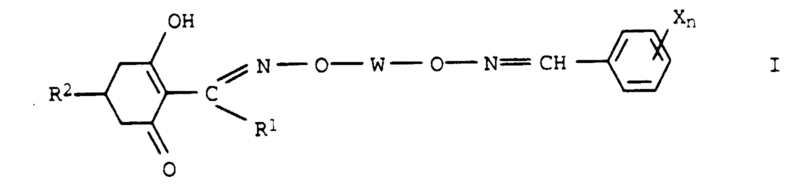 in der die Substituenten und der Index folgende Bedeutung haben:
- R¹: eine C₁-C₆-Alkylgruppe;
- W: eine gewünschtenfalls durch C₁-C₃-Alkyl substituierte C₂-C₄-Alkylenkette;
- X: Nitro, Cyano, Halogen, C₁-C₄-Alkyl, C₁-C₄-Halogenalkyl;
- n: 0 bis 3, wobei die Reste X verschieden sein können, wenn n 2 oder 3 bedeutet, oder 1 bis 5 für den Fall, daß alle X Halogen bedeuten;
- R²: eine C₁-C₄-Alkoxy-C₁-C₆-alkyl- oder C₁-C₄-AlkylthioC₁-C₆-alkylgruppe;
eine C₃-C₇-Cycloalkylgruppe oder eine C₅-C₇-Cycloalkenylgruppe, wobei diese Gruppen gewünschtenfalls ein bis drei Substituenten tragen können, ausgewählt aus einer Gruppe bestehend aus C₁-C₄-Alkyl, C₁-C₄-Alkoxy, C₁-C₄-Alkylthio, C₁-C₄-Halogenalkyl, Hydroxyl und Halogen;
ein 5-gliedriger gesättigter Heterocyclus, der ein oder zwei Sauerstoff- und/oder Schwefelatome als Heteroatome enthält und der gewünschtenfalls noch ein bis drei Substituenten tragen kann, ausgewählt aus einer Gruppe bestehend aus C₁-C₄-Alkyl, C₁-C₄-Alkoxy, C₁-C₄-Alkylthio und C₁-C₄-Halogenalkyl;
ein 6- oder 7-gliedriger Heterocyclus mit einem oder zwei nicht benachtbarten Sauerstoff- und/oder Schwefelatomen als Heteroatomen, der gesättigt oder ein- oder zweifach ungesättigt sein kann, wobei der Heterocyclus gewünschtenfalls noch ein bis drei Substituenten tragen kann, ausgewählt aus einer Gruppe bestehend aus Hydroxyl, Halogen, C₁-C₄-Alkyl, C₁-C₄-Alkoxy, C₁-C₄-Alkylthio und C₁-C₄-Halogenalkyl;
ein 5-gliedriger Heteroaromat, enthaltend ein bis zwei Stickstoffatome und ein Sauerstoff- oder Schwefelatom, wobei dieser Ring gewünschtenfalls noch ein bis drei Substituenten tragen kann, ausgewählt aus einer Gruppe bestehend aus Halogen, Cyano, C₁-C₄-Alkyl, C₁-C₄-Alkoxy, C₁-C₄-Alkylthio, C₁-C₄-Halogenalkyl, C₂-C₆-Alkenyl, C₂-C₆-Alkenyloxy und C₁-C₄-Alkoxy-C₁-C₄-alkyl;
eine Phenyl- oder Pyridylgruppe, wobei diese Gruppen gewünschtenfalls noch ein bis drei der folgenden Substituenten tragen können ausgewählt aus einer Gruppe bestehend aus Halogen, Nitro, Cyano, C₁-C₄-Alkyl, C₁-C₄-Alkoxy, C₁-C₄-Alkylthio, C₁-C₄-Halogenalkyl, C₃-C₆-Alkenyloxy, C₃-C₆-Alkinyloxy und eine Aminogruppe -NR^{a}R^{b}, worin
R^{a} für Wasserstoff, C₁-C₄-Alkyl, C₃-C₆-Alkenyl oder C₃-C₆-Alkinyl steht und
R^{b} für Wasserstoff, C₁-C₄-Alkyl, C₃-C₆-Alkenyl, C₃-C₆-Alkinyl oder C₁-C₆-Acyl steht oder für Benzoyl steht, das gewünschtenfalls seinerseits noch ein bis drei Reste tragen kann, ausgewählt aus einer Gruppe bestehend aus Nitro, Cyano, Halogen, C₁-C₄-Alkyl, C₁-C₄-Alkoxy, C₁-C₄-Alkylthio und C₁-C₄-Halogenalkyl;
sowie die landwirtschaftlich brauchbaren Salze und Ester von C₁-C₁₀-Carbonsäuren und anorganischen Säuren der Verbindungen I.

Außerdem betrifft die Erfindung ein Verfahren zur Herstellung dieser Verbindungen, ihre Verwendung als Herbizide sowie herbizide Mittel, welche diese Verbindungen als wirksame Substanzen enthalten.

Des weiteren betrifft die Erfindung neue Hydroxylamine der Formel III 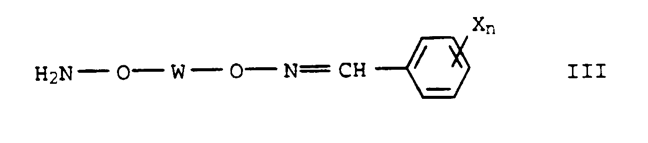 wobei
- W: für eine gewünschtenfalls durch C₁-C₃-Alkyl substituierte C₂-C₄-Alkylenkette;
- X: für Nitro, Cyano, Halogen, C₁-C₄-Alkyl, C₁-C₄-Halogen-alkyl und
- n: für 0 bis 3, wobei die Reste X verschieden sein können, wenn n 2 oder 3 bedeutet, oder 1 bis 5 für den Fall, daß alle X Halogen bedeuten, stehen.

Aus der Literatur sind bereits herbzid wirksame Cyclohexandione der Formel I' 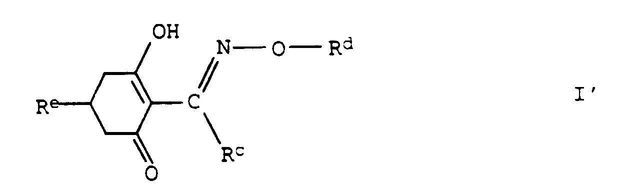 bekannt, wobei R^{c}, R^{d} und R^{e} u. a. für die folgenden Bedeutungen stehen:
- US 4,440,566 (R^{c} = Ethyl, Propyl; R^{d} = Benzyl; R^{e} = 2-Ethylthiopropyl);
- EP-A 238 021 und EP-A 125 094 (R^{c} = Ethyl, Propyl; R^{d} = Benzyl, But-2-enyl; R^{e} = substituierter 5-gliedriger Heteroarylrest);
- EP-A 80 301 (R^{c} = Ethyl, Propyl; R^{d} = Benzyl, But-2-enyl; R^{e} = substituiertes Phenyl);
- DE-A 38 38 309 (R^{c} = Ethyl, Propyl; R^{d} = substituierter 4-Phenylbutylen- oder 4-Phenylbutenylenrest; R^{e} = substituierter 5- bis 7-glieriger Heterocyclus);
- EP-A 456 112 (R^{c} = Ethyl, Propyl; R^{d} = substituierter 3-Phenoxypropylen- oder 2-Phenoxyethylenrest; R^{e} = substituierter 5- bis 7-gliedriger Heterocyclus).

Die herbiziden Eigenschaften dieser Verbindungen, insbesondere bezüglich ihrer Selektivität gegen Ungräser in grasartigen Kulturpflanzen, können jedoch nur bedingt befriedigen.

Der Erfindung lagen daher neue Cyclohexenonoximether mit verbesserter Selektivität gegen Ungräser in grasartigen Kulturen wie Reis und Mais zugrunde.

Demgemäß wurden die eingangs definierten Cyclohexenonoximether I gefunden. Außerdem wurden herbizide Mittel gefunden, die diese Substanzen enthalten.

Die Cyclohexenonoximether I sind auf verschiedene Weise erhältlich, und zwar bevorzugt in an sich bekannter Weise aus schon bekannten Cyclohexenonen der Formel II, (DE-A 38 38 309, EP-A 456 112) und den neuen Hydroxylaminen der Formel III:

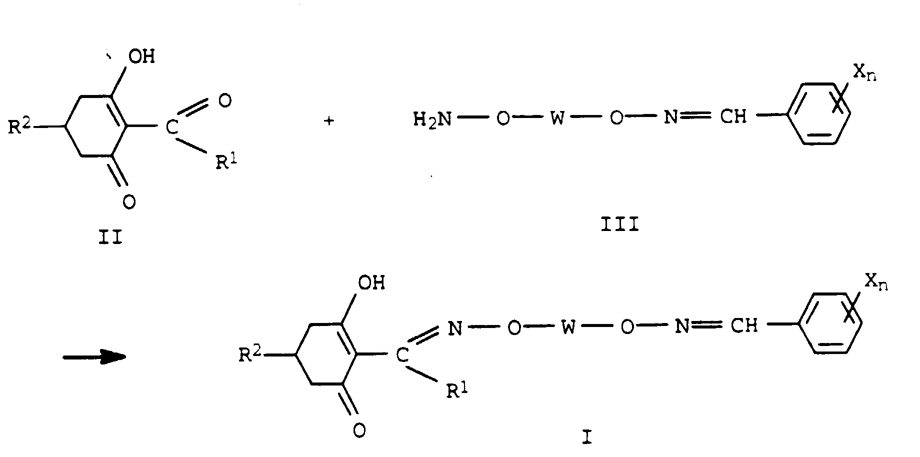

Vorzugsweise verwendet man ein geeignetes Salz des Hydroxylamins III, insbesondere dessen Hydrochlorid, und führt die Reaktion in heterogener Phase in einem inerten Lösungsmittel durch, beispielsweise in Dimethylsulfoxid, in einem Alkohol wie Methanol, Ethanol und Isopropanol, in einem aromatischen Kohlenwasserstoff wie Benzol und Toluol, in einem chlorierten Kohlenwasserstoff wie Chloroform und 1,2-Dichlorethan, in einem aliphatischen Kohlenwasserstoff wie Hexan und Cyclohexan, in einem Ester wie Essigsäureethylester oder in einem Ether wie Diethylether, Dioxan und Tetrahydrofuran.

Die Reaktionsführung erfolgt in Gegenwart einer Base, wobei normalerweise eine Basenmenge von etwa 0,5 bis 2 Mol-Äquivalent, bezogen auf die Ammoniumverbindung, ausreichend ist.

Als Basen kommen z.B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid oder Calciumoxid in Betracht. Des weiteren sind organische Basen wie Pyridin und tert. Amine wie Triethylamin geeignet.

Vorzugsweise führt man die Umsetzung in Methanol mit Natriumhydrogencarbonat als Base durch.

Eine Variante des Verfahrens besteht darin, die Umsetzung ohne Base mit der freien Hydroxylaminbase III, z. B. in Form einer wäßrigen Lösung, vorzunehmen; je nach verwendetem Lösungsmittel für die Verbindung II erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol, aliphatische und aromatische, gegebenenfalls chlorierte Kohlenwasserstoffe wie Hexan, Cyclohexan, Methylenchlorid, Toluol und Dichlorethan, Ester wie Essigsäureethylester, Nitrile wie Acetonitril und cyclische Ether wie Dioxan und Tetrahydrofuran.

Zweckmäßigerweise setzt man das Cyclohexenon II und das Hydroxylamin III bzw. dessen Salz in etwa stöchiometrischem Verhältnis ein, jedoch kann in manchen Fällen auch ein Überschuß der einen oder anderen Komponente, bis ca. 10 mol-%, vorteilhaft sein.

Die Reaktionstemperatur liegt im allgemeinen zwischen 0°C und dem Siedepunkt des Reaktionsgemisches, vorzugsweise zwischen 20 und 80°C.

Die Reaktion ist nach wenigen Stunden beendet. Das Produkt kann auf übliche Weise, z. B. durch Einengen der Mischung, Verteilung des Rückstandes in Methylenchlorid/Wasser und Abdestillieren des Lösungsmittels unter vermindertem Druck, isoliert werden.

Besondere Bedingungen bezüglich des Drucks sind nicht zu beachten; im allgemeinen arbeitet man daher bei Normaldruck oder unter dem Eigendruck des jeweiligen Verdünnungsmittels.

Aufgrund ihres sauren Charakters können die erfindungsgemäßen Cyclhexenonoximether I Salze von Alkali- oder Erdalkalimetallverbindungen sowie Enolester bilden.

Alkalimetallsalze der Verbindungen I können durch Behandeln der 3-Hydroxycyclohexenon-Verbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Phosphonium-, Sulfonium- oder Sulfoxoniumnydroxiden.

Die Ester der Verbindungen I sind ebenfalls in üblicher weise erhältlich (vgl. z.B. Organikum, VEB Deutscher Verlag der Wissenschaften, 17. Auflage, Berlin 1988, S. 405-408).

Die neuen Hydroxylamine der Formel III 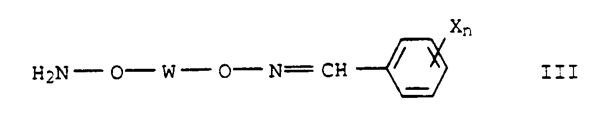 wobei
- W: für eine gewünschtenfalls durch C₁-C₃-Alkyl substituierte C₂-C₄-Alkylenkette;
- X: für Nitro, Cyano, Halogen, C₁-C₄-Alkyl, C₁-C₄-Halogenalkyl, und
- n: für 0 bis 3, wobei die Reste X verschieden sein können, wenn n 2 oder 3 bedeutet, oder 1 bis 5 für den Fall, daß alle X Halogen bedeuten,
stehen, lassen sich in der Regel über eine Reihe bekannter Verfahrensschritte ausgehend von bekannten Vorprodukten herstellen. Vorzugsweise koppelt man ein 1,ω-Bis-(aminooxy)alkan IV (Angew. Makromol. Chem. 184, 125 (1985); Bioorg. Khim. 11, 1574 (1985); J. Org. Chem. 54, 2351 (1989) auf für Hydroxylamine bekannte Weise (Houben-Weyl, Methoden der organischen Chemie, Bd. E 14b, S. 369) unter Wasserabspaltung mit einem Aldehyd V zum Hydroxylaminderivat III:

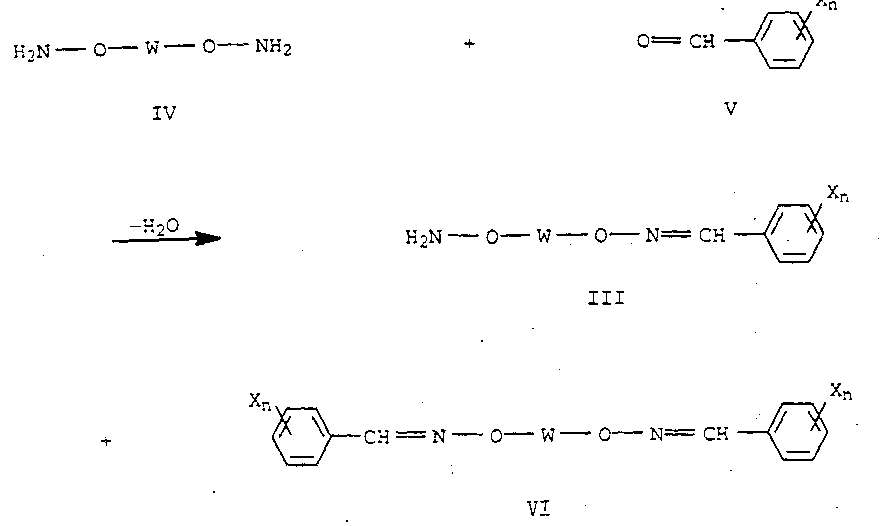

Als Nebenprodukte treten die 1,ω-Bis(benzylideniminooxy)alkane VI auf.

Aus den nach diesen Verfahren erhaltenen Reaktionsgemischen können die Hydroxylamine III mittels üblicher Aufarbeitungsmethoden isoliert weren, beispielsweise durch Extraktion oder durch Kristallisation. Zur Erhöhung der Kristallisationstendenz der Hydroxylaminderivate kann es vorteilhaft sein, sie in die Salze von Mineralsäuren oder von organischen Säuren zu überzuführen. Dazu werden im allgemeinen verdünnte Lösungen dieser Säuren mit den Hydroxylaminen III umgesetzt, und zwar zweckmäßigerweise ca. äquivalente Mengen an Säure und Hydroxylamin III.

Die erhaltenen Hydroxylammoniumsalze können wie die Hydroxylamine III mit freier Aminogruppe direkt zu den Herbiziden der Formel I weiterverarbeitet werden oder auch, falls gewünscht, gelagert werden.

Die Cyclohexenonoximether I können bei der Herstellung als Isomerengemische anfallen, wobei sowohl E-/Z-Isomerengemische als auch Enantiomeren- oder Diastereoisomerengemische möglich sind. Die Isomerengemische können gewünschtenfalls nach den hierfür üblichen Methoden, z. B. durch Chromatographie oder durch Kristallisation, getrennt werden.

Die Cyclohexenonoximether I können in mehreren tautomeren Formen geschrieben werden, die alle von der Erfindung umfaßt werden.

Die in den Definitionen der Substituenten verwendeten Sammelbegriffe
- Halogen,
- C₁-C₄-Alkyl, C₁-C₄-Alkoxy, C₁-C₄-Alkylthio, C₁-C₄-Halogenalkyl,
- C₂-C₆-Alkenyl, C₂-C₆-Alkenyloxy,
- C₃-C₆-Alkenyl, C₃-C₆-Alkenyloxy, C₃-C₆-Alkinyl, C₃-C₆-Alkinyloxy,
- C₁-C₆-Acyl
stellen Kurzschreibweisen für eine individuelle Aufzählung der einzelnen Gruppenmitglieder dar. Sämtliche Alkyl-, Alkoxy-, Alkylthio-, Halogenalkyl-, Alkenyl-, Alkenyloxy-, Alkinyl- und Alkinyloxyteile können geradkettig oder verzweigt sein. Die Halogenalkylteile können gleiche oder verschiedene Halogenatome tragen.

Im einzelnen bedeuten beispielsweise
- Halogen: Fluor, Chlor, Brom und Jod;
- C₁-C₄-Alkyl: Methyl, Ethyl, n-Propyl, 1-Methylethyl, n-Butyl, 1-Methylpropyl, 2-Methylpropyl und 1,1-Dimethylethyl;
- C₁-C₄-Alkoxy: Methoxy, Ethoxy, n-Propoxy, 1-Methylethoxy, n-Butoxy, 1-Methylpropoxy, 2-Methylpropoxy und 1,1-Dimethylethoxy;
- C₁-C₄-Alkylthio: Methylthio, Ethylthio, n-Propylthio, 1-Methylethylthio, n-Butylthio, 1-Methylpropylthio, 2-Methylpropylthio und 1,1-Dimethylethylthio;
- C₁-C₄-Halogenalkyl: Fluormethyl, Difluormethyl, Trifluormethyl, Chlordifluormethyl, Dichlorfluormethyl, Trichlormethyl, 1-Fluorethyl, 2 -Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl;
- C₂-C₆-Alkenyl: Ethenyl und C₃-C₆-Alkenyl wie 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
- C₂-C₆-Alkenyloxy: Ethenyloxy und C₃-C₆-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyl-oxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy;

Im Hinblick auf die biologische Wirksamkeit werden Cyclohexenone der Formel I bevorzugt, in denen die Substituenten folgende Bedeutung haben:
- R¹: C₁-C₆-Alkyl wie Methyl, Ethyl, n-Propyl, n-Butyl, n-Pentyl, n-Hexyl, insbesondere Ethyl und Propyl;
- W: C₂-C₄-Alkylen wie Ethylen, Propylen, Butylen, die durch ein bis drei Methyl- bzw. Ethylreste substituiert sein können. Besonders bevorzugt sind Ethylen, Propylen, 2-Methylpropylen, 2,2-Dimethylpropylen und Butylen;
- X: - Nitro, Cyano,
- Halogen, insbesondere Fluor, Chlor und Brom;
- C₁-C₄-Alkyl, insbesondere Methyl;
- C₁-C₄-Halogenalkyl, insbesondere Difluormethyl, Trifluormethyl, 2,2,2-Trifluorethyl und Pentafluorethyl; besonders bevorzugt ist Halogen;
- n: 0 bis 3, wobei die Reste X verschieden sein können, wenn n 2 oder 3 bedeutet, oder 1 bis 5 für den Fall, daß alle X Halogen bedeuten; besonders bevorzugt bedeutet n 1 bis 3;
- R²: - eine C₁-C₆-Alky lgruppe wie Methyl, Ethyl, Propyl, 1-Methylethyl, n-Butyl, 1-Methylpropyl, 2-Methylpropyl und 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethybutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und l-Ethyl-2-methylpropyl, wobei die Alkylgruppe durch C₁-C₄-Alkoxy, insbesondere Methoxy, Ethoxy, 1-Methylethoxy und 1,1-Dimethylethoxy, oder durch C₁-C₄-Alkylthio, insbesondere Methylthio und Ethylthio, substituiert ist, und zwar bevorzugt in 1-, 2- oder 3-Position; ganz besonders bevorzugt ist 2-Ethylthiopropyl;
- eine C₃-C₇-Cycloalkylgruppe oder eine C₅-C₇-Cycloalkenylgruppe wie Cyclopropyl, Cyclobutyl, Cyclopentyl, Cyclohexyl,Cycloheptyl, Cyclopentenyl, Cyclohexenyl und Cycloheptenyl,wobei diese Gruppen ein bis drei der folgenden Substituententragen können: C₁-C₄-Alkyl, C₁-C₄-Alkoxy, C₁-C₄-Alkylthio und C₁-C₄-Halogenalkyl; ganz besonders bevorzugt ist 1-Methylthio-1-cyclopropyl;
- ein 5-gliedriger gesättigter Heterocyclus wie Tetrahydrofuranyl, Tetrahydrothienyl, Dioxolanyl, Dithiolanyl und Oxathiolanyl, insbesondere Tetrahydrofuranyl, Tetrahydrothienyl und Dioxolanyl, wobei diese Ringe unsubstituiert sein oder ein bis drei Substituenten tragen können, ausgewählt aus einer Gruppe bestehend aus C₁-C₄-Alkyl, C₁-C₄-Alkoxy, C₁-C₄-Alkylthio und C₁-C₄-Halogenalkyl;
- ein 5-gliedriger Heteroaromat wie Pyrrolyl, Pyrazolyl, Imidazolyl, Isoxazolyl, Oxazolyl, Isothiazolyl, Thiazolyl, Furanyl und Thienyl, insbesondere Isoxazolyl und Furanyl, wobei der 5-gliedrige Heteroaromat unsubstituiert sein oder ein bis drei Substituenten tragen kann, ausgewählt aus einer Gruppe bestehend aus C₁-C₄-Alkyl, C₁-C₄-Alkoxy, C₁-C₄-Alkylthio, C₁-C₄-Halogenalkyl, C₁-C₄-Alkoxy-C₁-C₄-alkyl wie Methoxymethyl, 2-Methoxyethyl,2-Methoxypropyl, 3-Methoxypropyl, 2-Methoxy-1-methylethyl, Ethoxymethyl, 2-Ethoxyethyl, 2-Ethoxypropyl, 3-Ethoxypropyl,2-Ethoxy-1-methylethyl und 1-Ethoxy-1-methylethyl, insbesondere Methoxyethyl und Ethoxyethyl,
   C₂-C₆-Alkenyl wie Ethenyl und C₃-C₆-Alkenyl,
   C₂-C₆-Alkenyloxy wie Ethenyloxy und C₃-C₆-Alkenyloxy, insbesondere 1-Methylethen-1-yloxy;
- ein 6- oder 7-gliedriger Heterocyclus der
   a) gesättigt sein kann, z.B. Tetrahydropyran-3-yl, Tetrahydropyran-4-yl, Tetrahydrothiopyran-3-yl, Tetrahydrothiopyran-4-yl und Dioxepan-5-yl,
   b) ein- oder zweifach ungesättigt sein kann, z.B. Dihydropyran-3-yl, Dihydropyran-4-yl, Dihydrothiopyran-3-yl und Dihydrothiopyran-4-yl,
   wobei die Heterocyclen unsubstituiert sein oder ein bis drei Substituenten tragen können, ausgewählt aus einer Gruppe bestehend aus Hydroxyl, Halogen, C₁-C₄-Alkyl, C₁-C₄-Alkoxy, C₁-C₄-Alkylthio und C₁-C₄-Halogenalkyl; ganz besonders bevorzugt sind Tetrahydropyran-3-yl, Tetrahydropyran-4-yl und Tetrahydrothiopyran-3-yl;
- eine Phenyl- oder Pyridylgruppe, die beide unsubstituiert sein oder ein bis drei Substituenten tragen können, ausgewählt aus einer Gruppe bestehend aus C₁-C₄-Alkyl, C₁-C₄-Alkoxy,C₁-C₄-Alkylthio, C₁-C₄-Halogenalkyl, C₃-C₆-Alkenyloxy, C₃-C₆-Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-3-butinyloxy, 2-Methyl-3-butinyloxy, 1-Methyl-2-butinyloxy, 1,1-Dimethyl-2-propinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-1-methyl-2-propinyloxy, vorzugsweise 2-Propinyloxy und 2-Butinyloxy; einer der drei Substituenten am Phenyl- oder Pyridylring kann auch eine Aminogruppe -NR^{a}R^{b} sein, wobei
   R^{a} für
   Wasserstoff,
   C₁-C₄-Alkyl, insbesondere Methyl und Ethyl, C₃-C₆-Alkenyl, insbesondere 2-Propenyl und 2-Butenyl, C₃-C₆-Alkinyl, insbesondere 2-Propinyl und 2-Butinyl, und
   R^{b} für
   Wasserstoff,
   C₁-C₄-Alkyl, insbesondere Methyl und Ethyl, C₃-C₆-Alkenyl, insbesondere 2-Propenyl und 2-Butenyl, C₃-C₆-Alkinyl, insbesondere 2-Propinyl und 2-Butinyl, C₁-C₆-Acyl wie Acetyl, Propionyl, n-Butyryl, 2-Methylpropionyl, Pentanoyl, 2-Methylbutyryl, 3-Methylbutyryl, 2,2-Dimethylpropionyl, n-Hexanoyl, 2-Methylpentanoyl, 2-Methylpentanoyl, 4-Methylpentanoyl, 2,2-Dimethylbutyryl, 2,3-Dimethylbutyryl,3,3-Dimethylbutyryl und 2-Ethylbutyryl, insbesondere Acetyl und Propionyl, oder für Benzoyl, das seinerseits unsubstituiert sein oder ein bis drei Reste tragen kann, ausgewählt aus einer Gruppe bestehend aus Nitro, Cyano, Halogen, vorzugsweise Fluor, Chlor und Brom, C₁-C₄-Alkyl, vorzugsweise Methyl, C₁-C₄-Alkoxy, vorzugsweise Methoxy und Ethoxy, C₁-C₄-Alkylthio, vorzugsweise Methylthio,sowie C₁-C₄-Halogenalkyl, vorzugsweise Trifluormethyl; stehen.
   Als Salze der Verbindungen der Formel I kommen landwirtschaftlich brauchbare Salze, beispielsweise Alkalimetallsalze, insbesondere das Natrium- oder Kaliumsalz, Erdalkalisalze insbesondere das Calcium-, Magnesium- oder Bariumsalz, Mangan-, Kupfer-, Zink- oder Eisensalz sowie Ammonium-, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.
   Unter Estern von C₁-C₁₀-Carbonsäuren sind insbesondere C₁-C₆-Alkylcarbonsäuren wie Methylcarbonsäure (Essigsäure), Ethylcarbonsäure (Propionsäure), Propylcarbonsäure (Buttersäure), 1-Methylethylcarbonsäure (Isobuttersäure), Butylcarbonsäure, 1-Methylpropylcarbonsäure, 2-Methylpropylcarbonsäure, 1,1-Dimethylethylcarbonsäure, Pentylcarbonsäure, 1-Methylbutylcarbonsäure, 2-Methylbutylcarbonsäure, 3-Methylbutylcarbonsäure, 1,1-Dimethylpropylcarbonsäure, 1,2-Dimethylpropylcarbonsäure, 2,2-Dimethylpropylcarbonsäure, 1-Ethylpropylcarbonsäure, Benzoesäure sowie durch Halogen substituierte Benzoesäuren, Hexylcarbonsaure, 1-Methylpentylcarbonsäure, 2-Methylpentylcarbonsaure, 3-Methylpentylcarbonsäure, 4-Methylpentylcarbonsaure, 1,1-Dimethylbutylcarbonsäure, 1,2-Dimethylbutylcarbonsäure, 1,3-Dimethylbutylcarbonsäure, 2,2-Dimethylbutylcarbonsäure, 2,3-Dimethylbutylcarbonsäure, 3,3-Dimethylbutylcarbonsäure, 1-Ethylbutylcarbonsäure, 2-Ethylbutylcarbonsaure, 1,1,2-Trimethylpropylcarbonsäure, 1,2,2-Trimethylpropylcarbonsäure, 1-Ethyl-1-methylpropylcarbonsäure und 1-Ethyl-2-methylpropylcarbonsäure, zu verstehen.

### Herstellungsbeispiele

2-[1-[[2-(4-Fluorbenzylideniminooxy)ethoxy]imino]propyl]-3-hydroxy-5-(2H-tetrahydrothiopyran-3-yl)-2-cyclohexen-1-on

Eine Mischung aus 3,0 g (11 mmol) 3-Hydroxy-2-propionyl-5-(2H- tetrahydrothiopyran-3-yl)-2-cyclohexen-1-on, 2,6 g (13 mmol) O-[2-(4-Fluorbenzylideniminooxy)ethyl]hydroxylamin und 100 ml Methanol wurde 16 Std. bei 25°C gerührt. Man engte unter reduziertem Druck ein und nahm den Rückstand in 100 ml 10 gew.-%iger Natronlauge auf. Die wässrige Phase wurde dreimal mit je 50 ml Methylenchlorid extrahiert, auf 0°C gekühlt und mit konz. Salzsäure auf pH 1 angesäuert. Anschließend extrahierte man mit 100 ml Diethylether, wonach die Etherphase über Natriumsulfat getrocknet, dann über Kieselgel filtriert und eingeengt wurde. Ausbeute: 45 %;
¹H-NMR (200 MHz, CDCl₃): δ = 1,15 ppm (t,3H);
1,50 - 2,60 ppm (m,15H); 2,85 ppm (m,2H); 4,35 ppm (m,4H); 7,00 ppm (m,2H); 7,55 ppm (m,2H); 8,05 ppm (s,1H).

### Vorstufe:

O-[2-(4-Fluorbenzylideniminooxy)ethyl]hydroxylamin

Zu einer Lösung aus 46 g (0,5 mol) 1,2-Bis(aminooxy)ethan in 50 ml Methanol tropfte man bei 23 - 35°C innerhalb von 2 Std. 50 g (0,4 mol) 4-Fluorbenzaldehyd in 200 ml Methanol und rührte 12 Std. bei 20 - 25° C nach. Nach Filtration*⁾ wurde die erhaltene Lösung eingeengt. Der Rückstand wurde in 100 ml Diethylether aufgenommen, wonach die organische Phase mit Wasser gewaschen, über Natriumsulfat getrocknet, über Kieselgel filtriert und schließlich erneut eingeengt wurde. Ausbeute: 56 %;
¹H-NMR (200 MHz, in CDCl₃): δ = 3,93 ppm (m,2H), 4,35 ppm (m,2H), 5,50 ppm (bs,2H); 7,00 ppm (m,2H); 7,45 ppm (m,2H); 8,10 ppm (s,1H).
*) Der bei der Reaktion ausgefallene Feststoff wurde mit Methanol und Petrolether gewaschen und anschließend getrocknet. Man erhielt 12 g 1,2-Bis-(4-fluorbenylideniminooxy)ethan; Schmp.: 83 - 85°C.

In der folgenden Tabelle 1 sind weitere Hydroxylamine III aufgeführt, die auf die gleiche Weise hergestellt wurden oder herstellbar sind. Die Tabellen 2 bis 5 enthalten erfindungsgemäße Cyclohexenonoximether I.

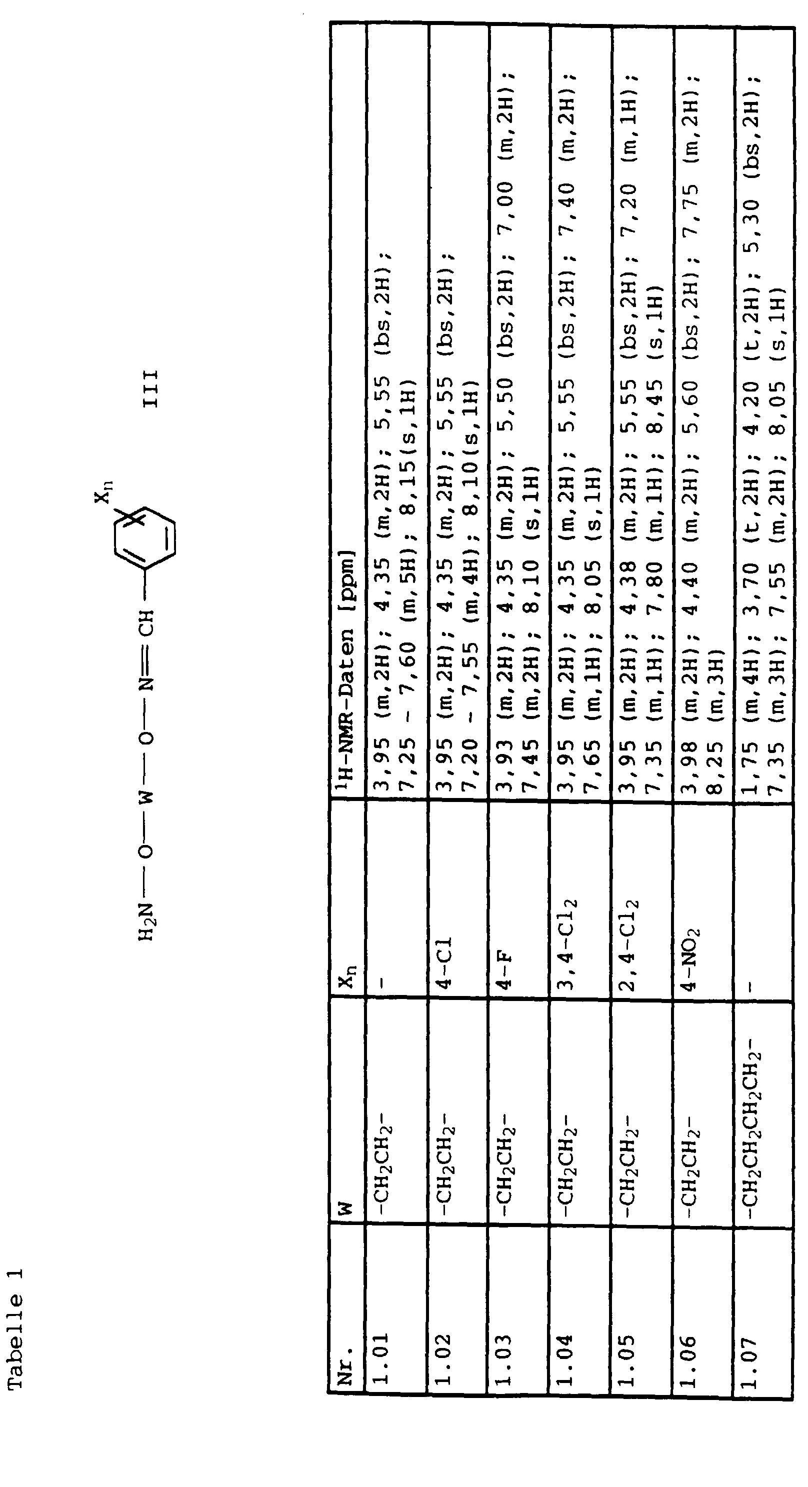

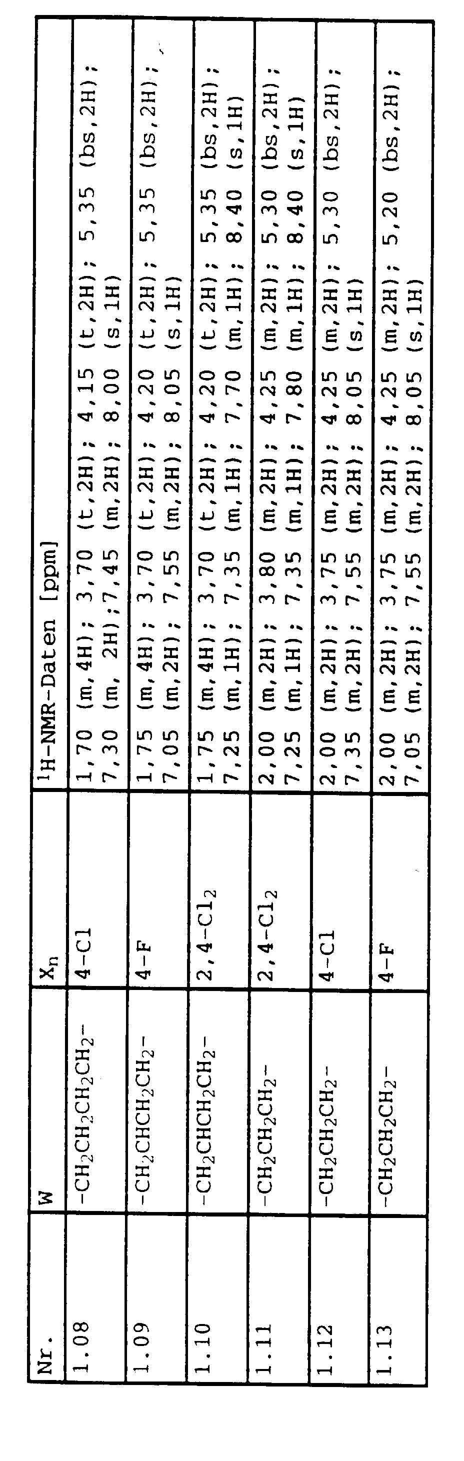

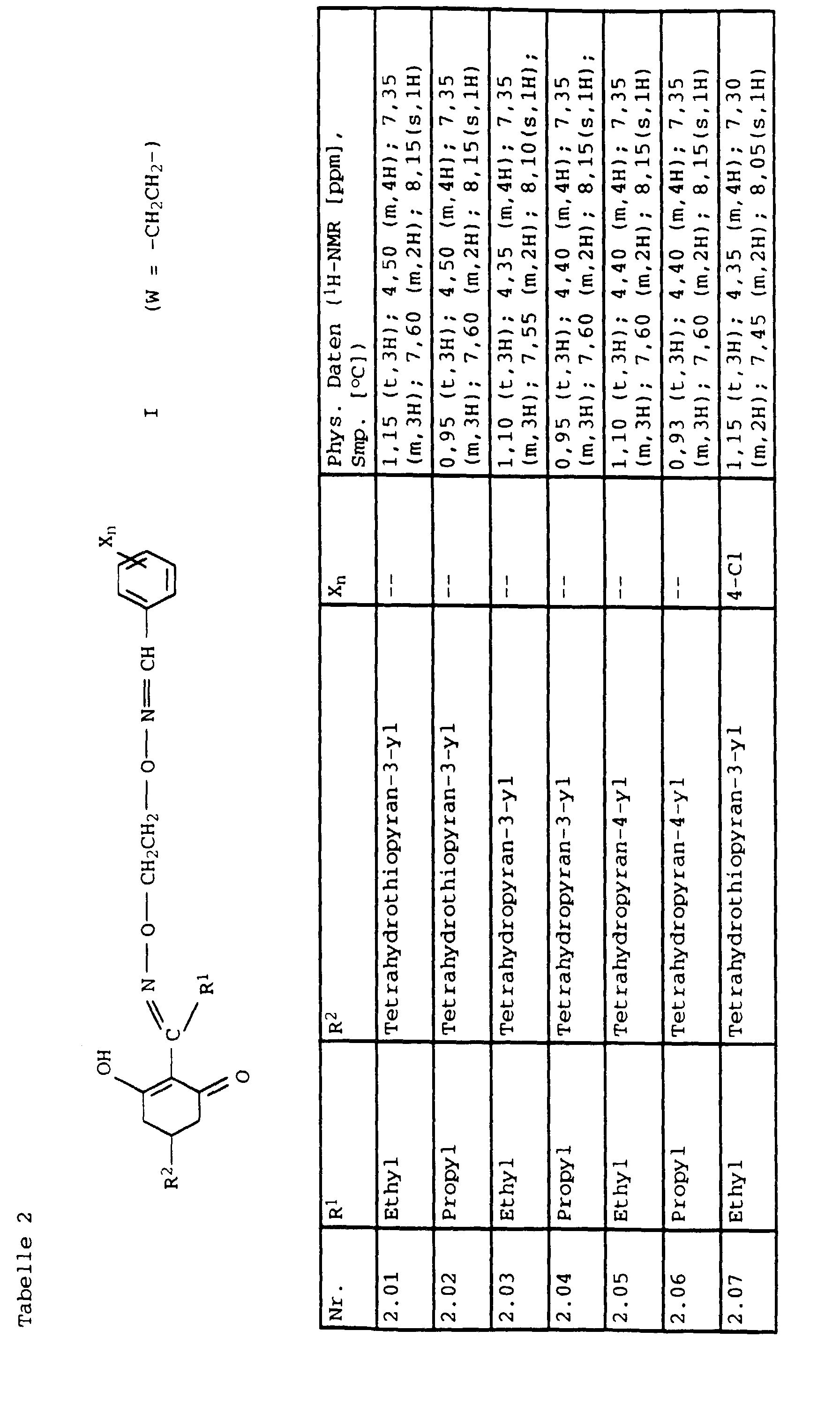

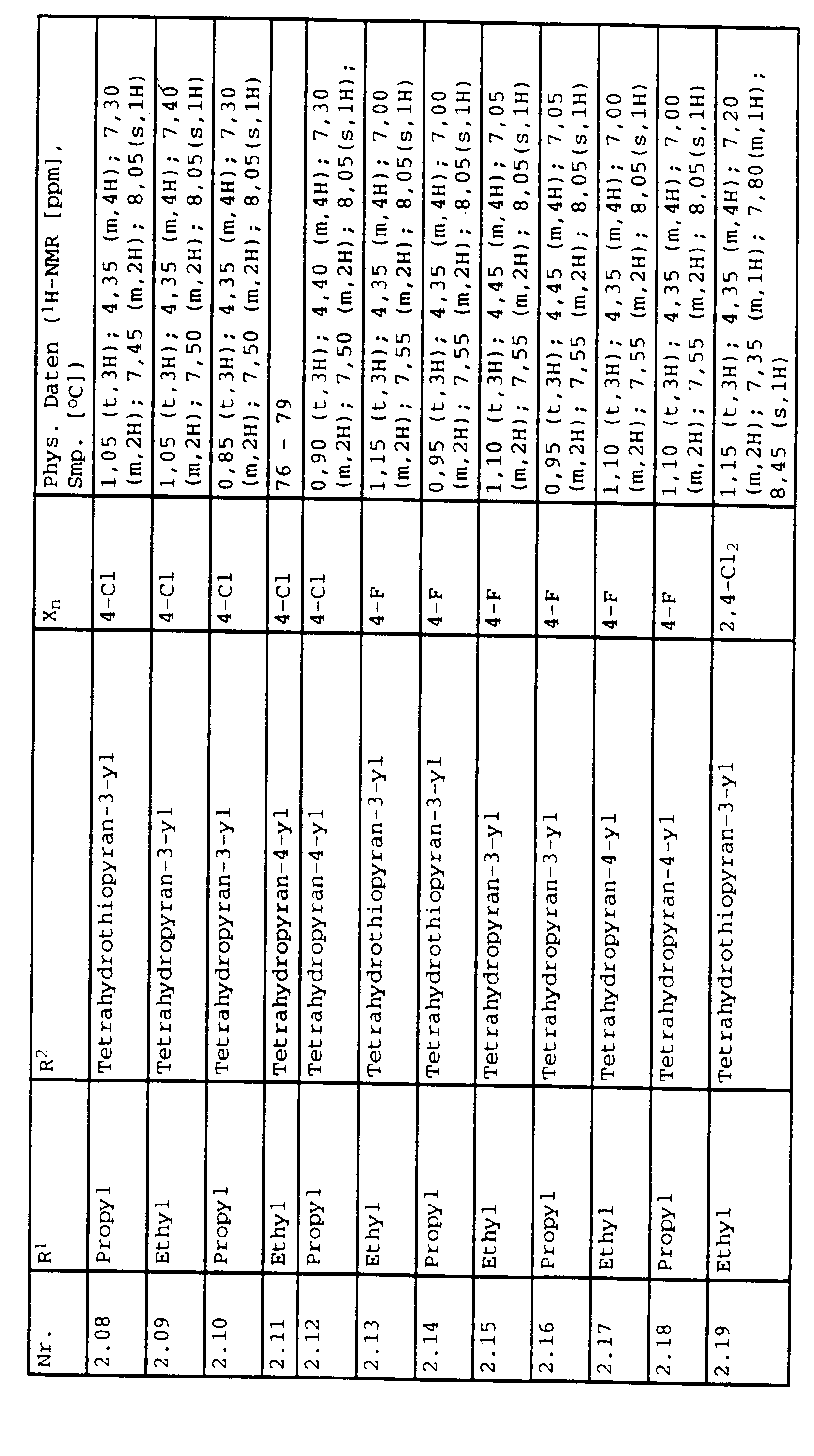

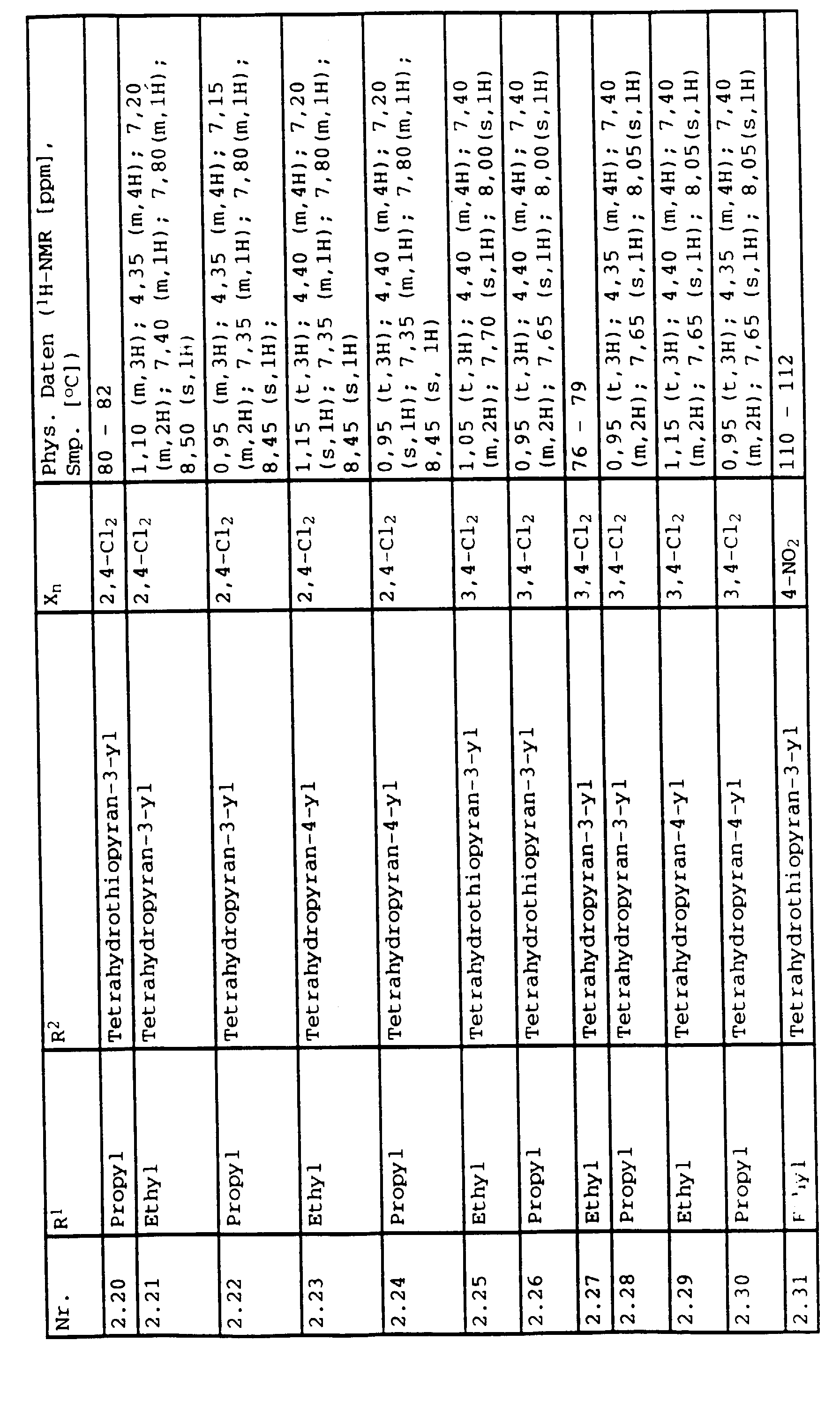

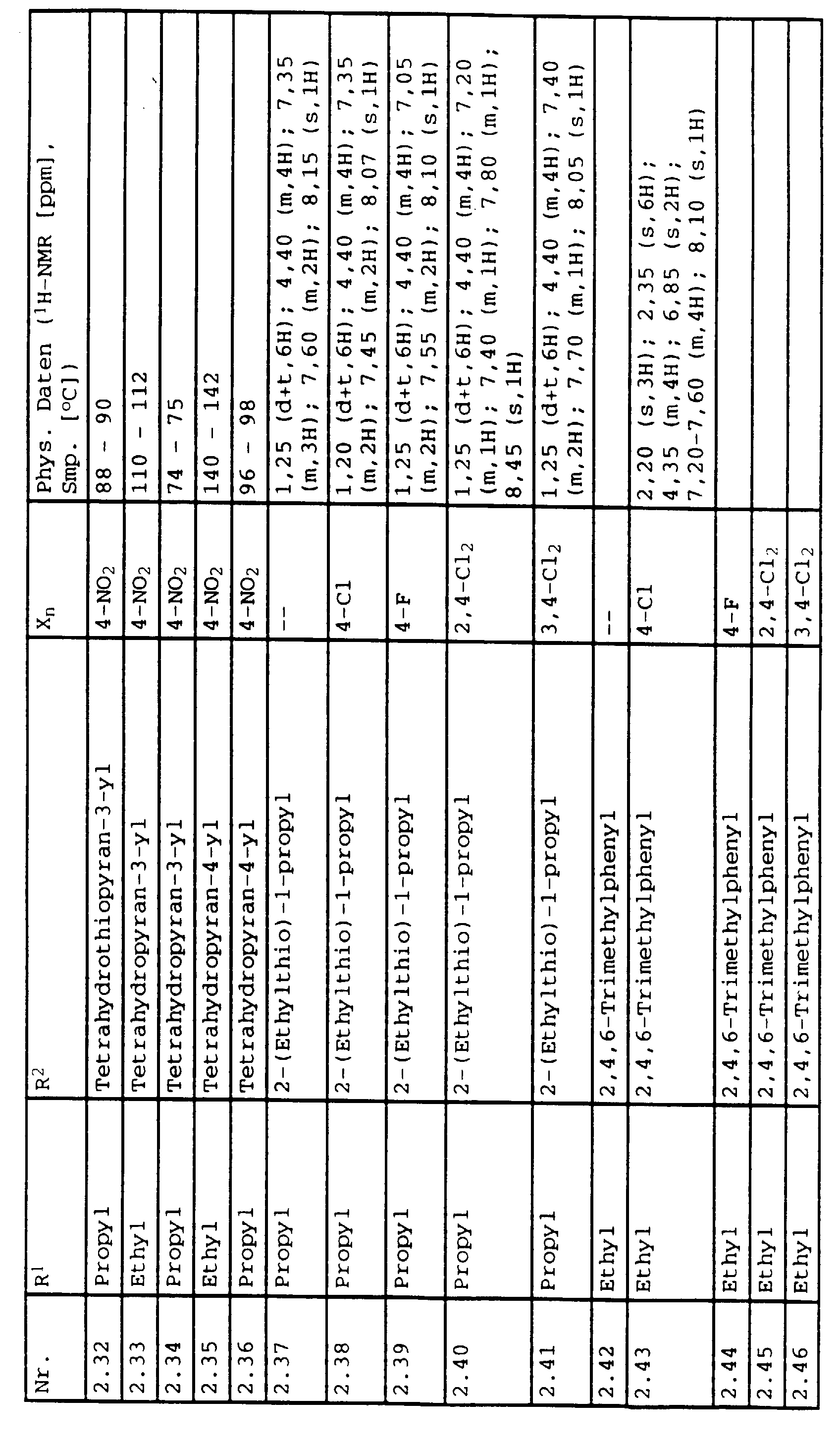

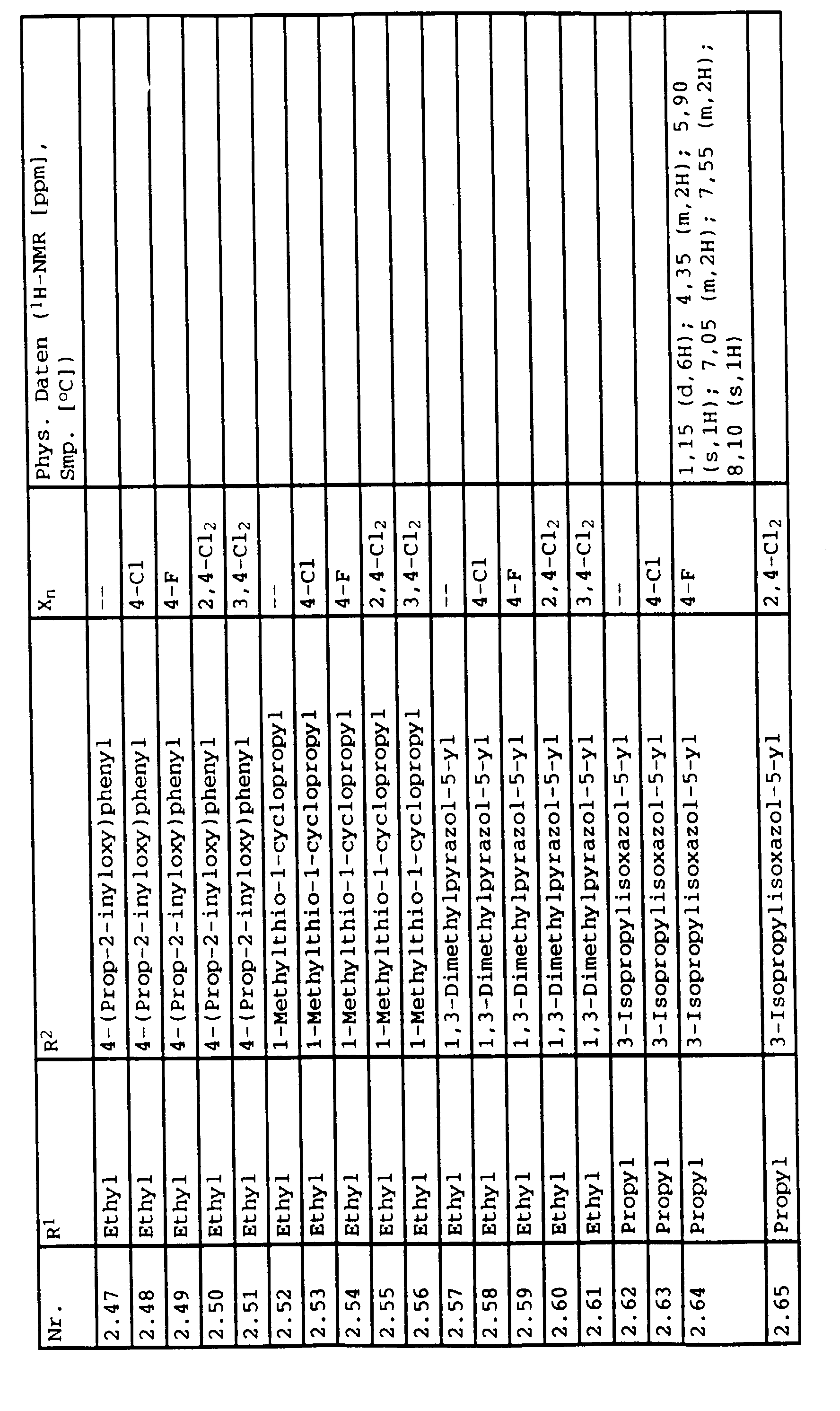

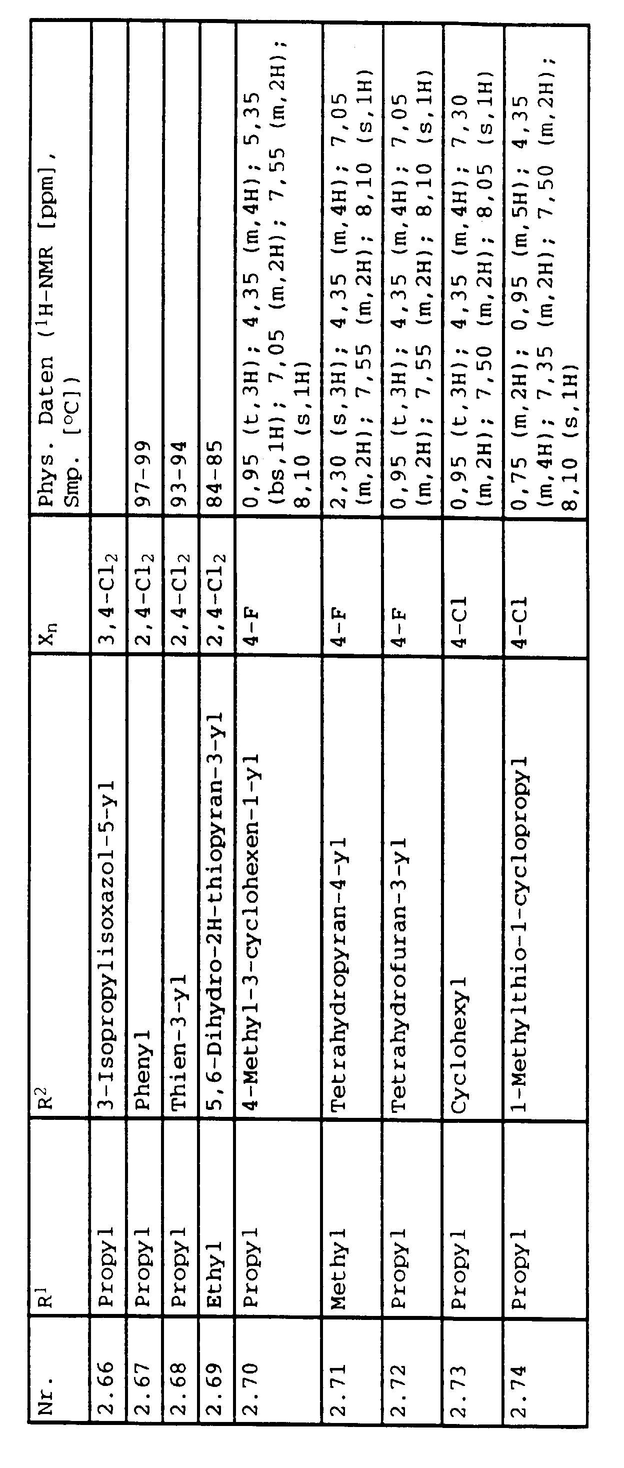

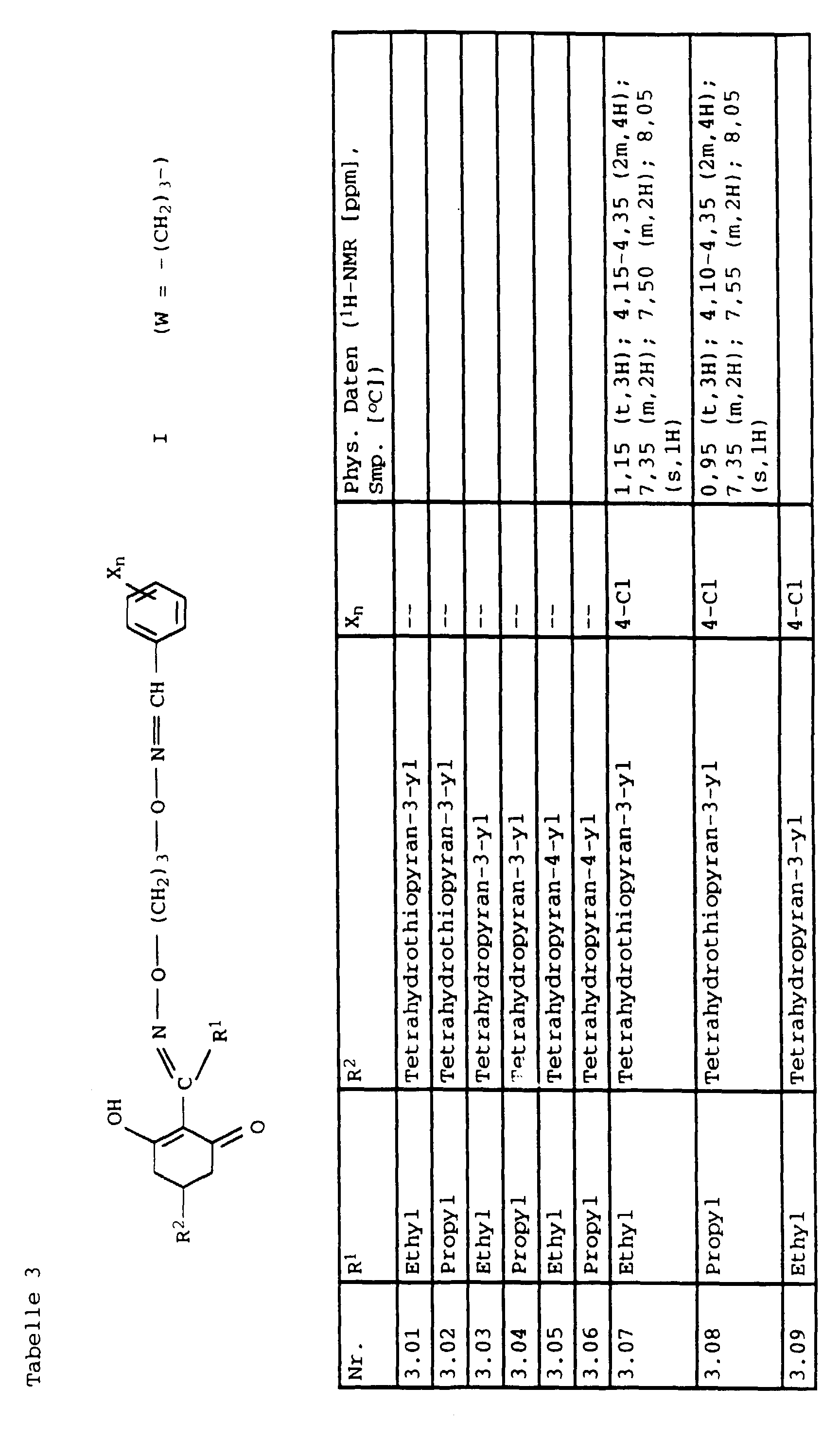

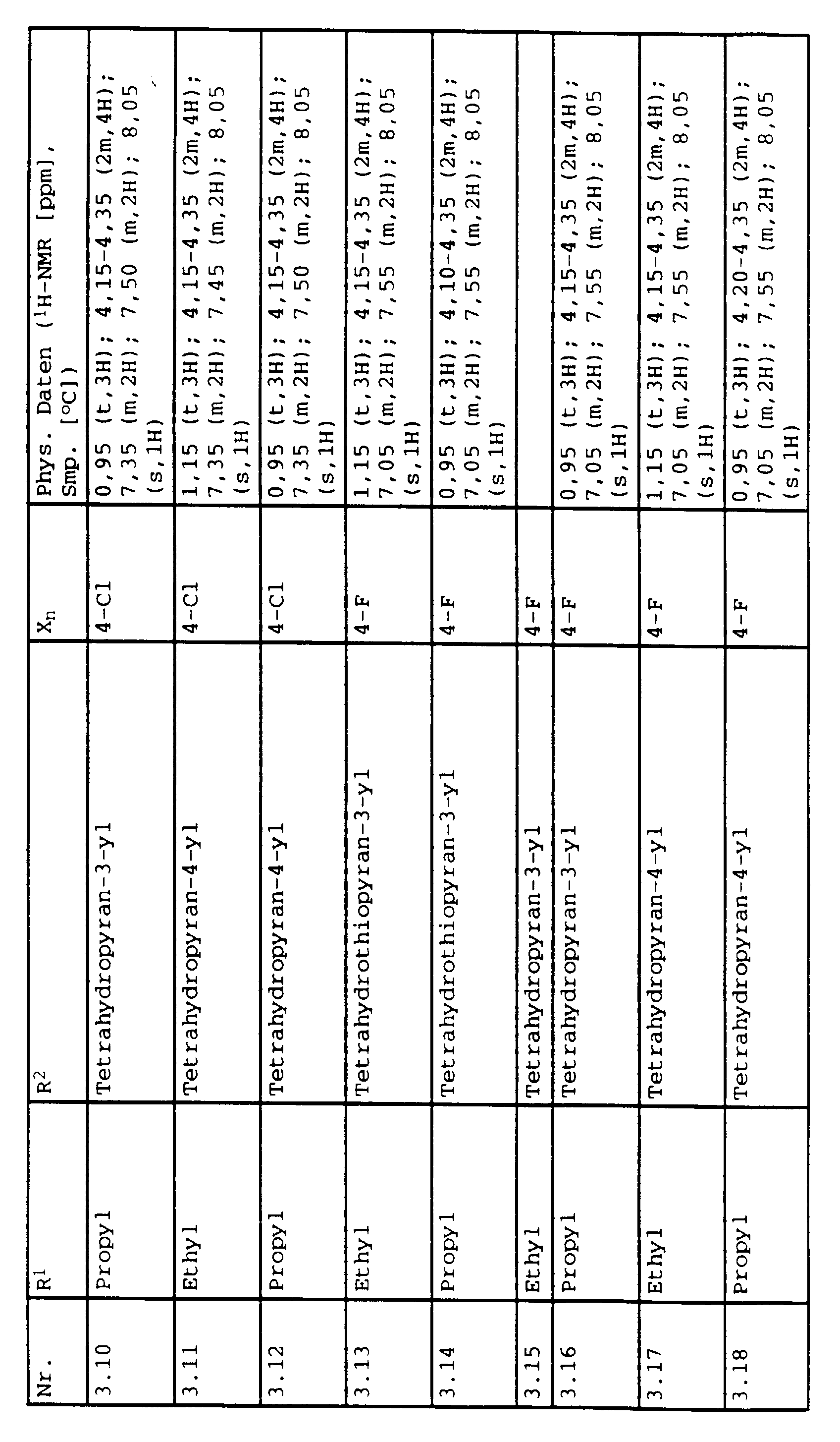

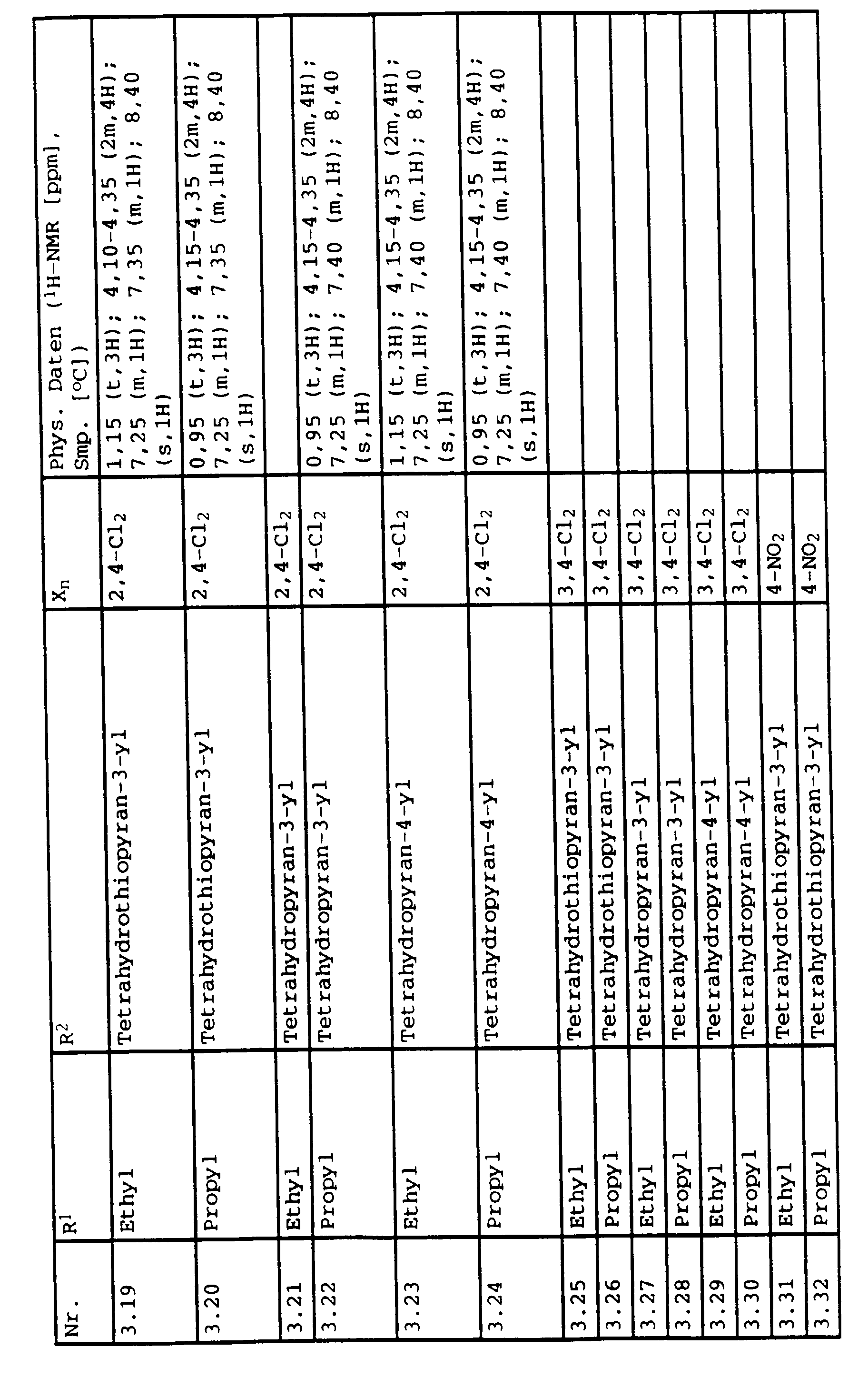

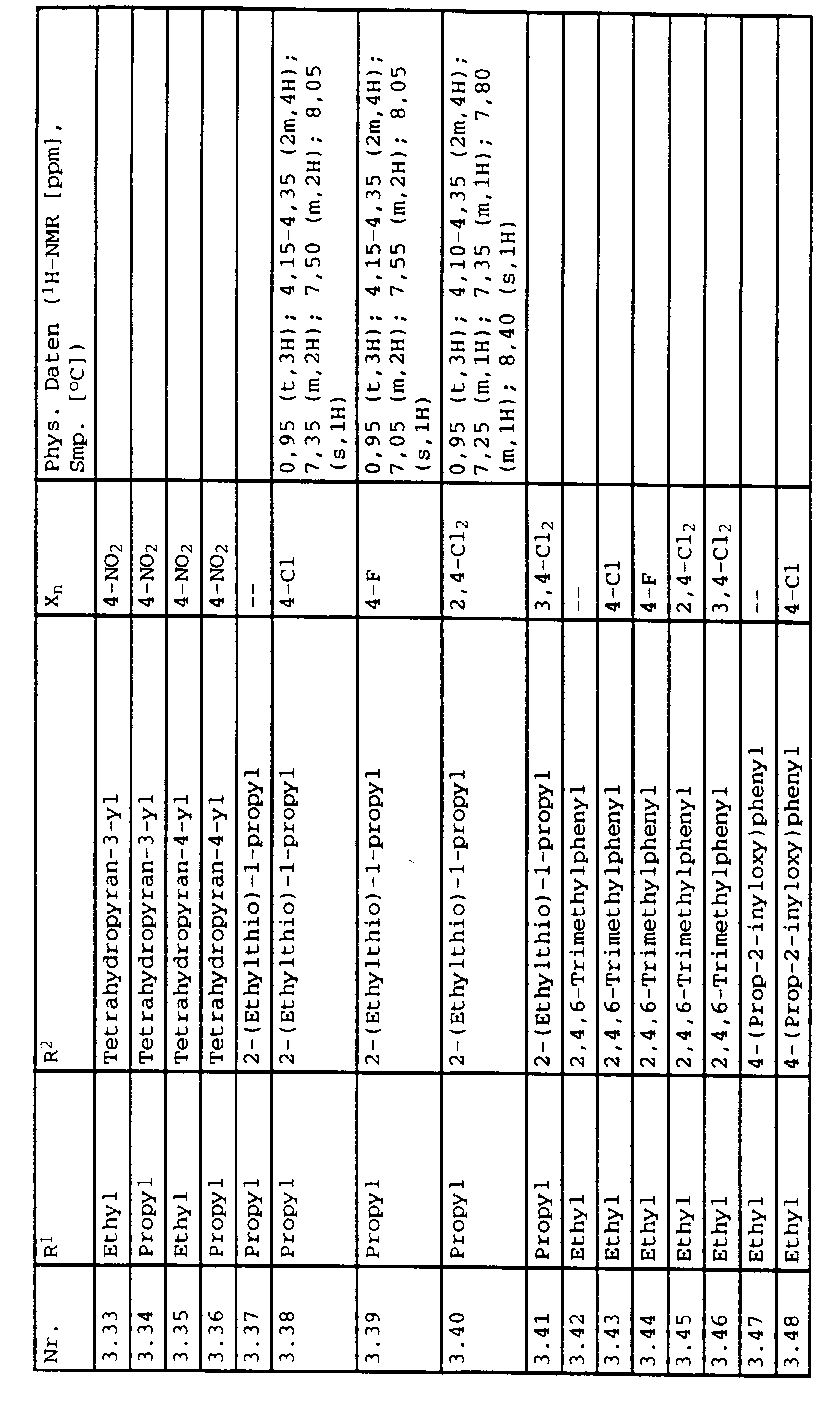

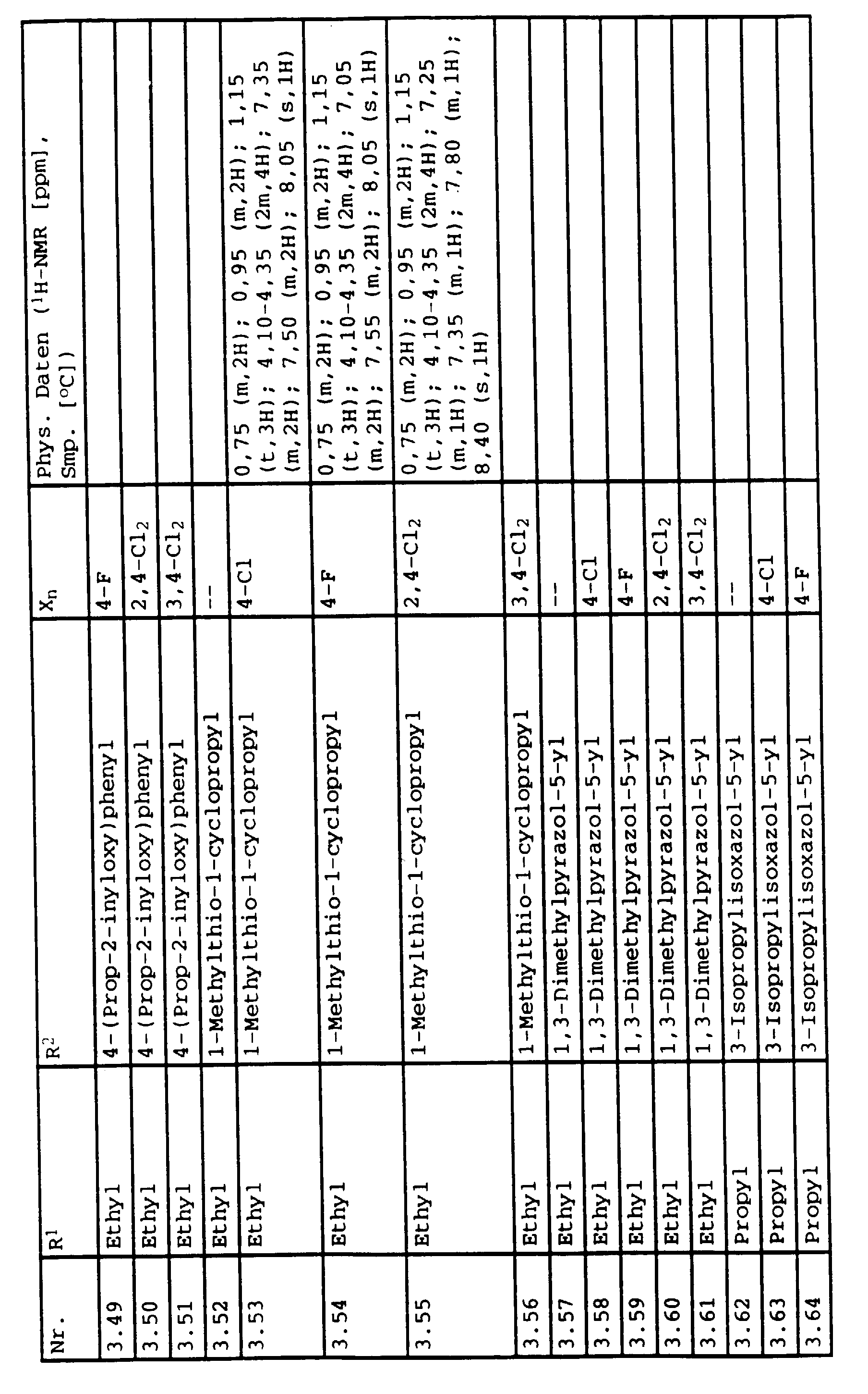

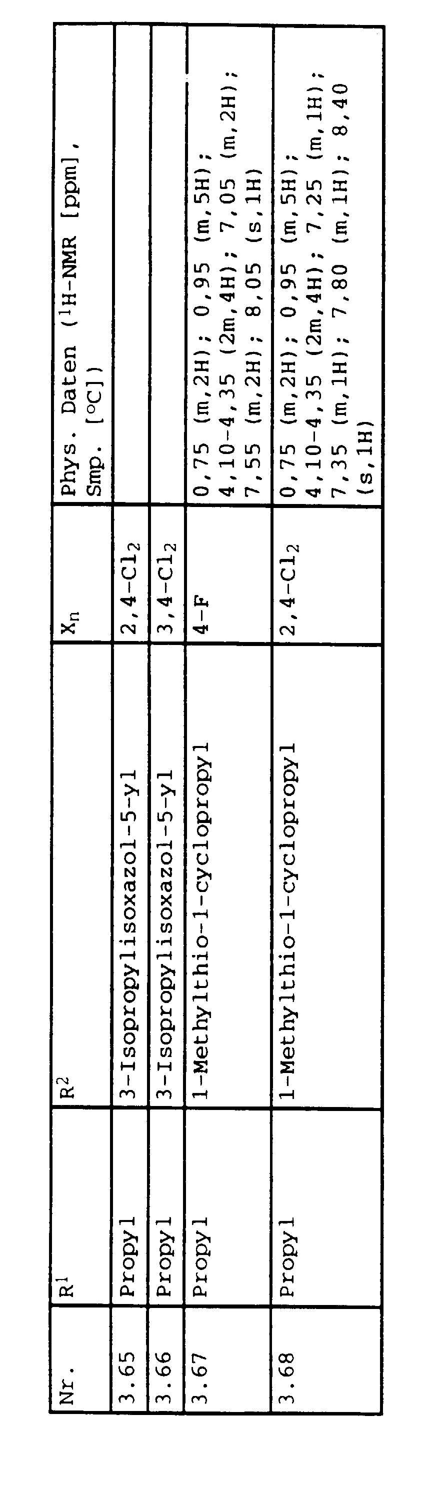

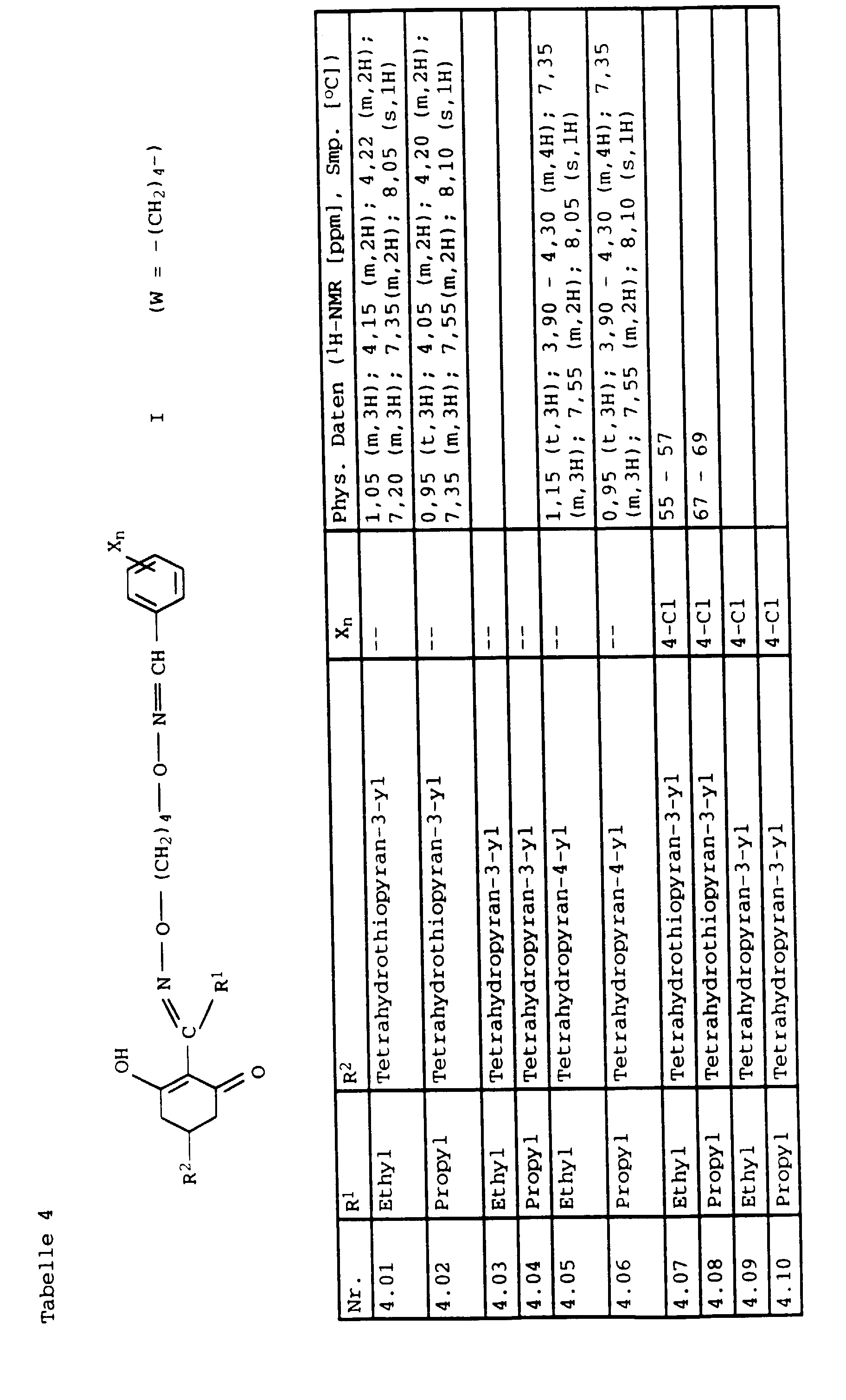

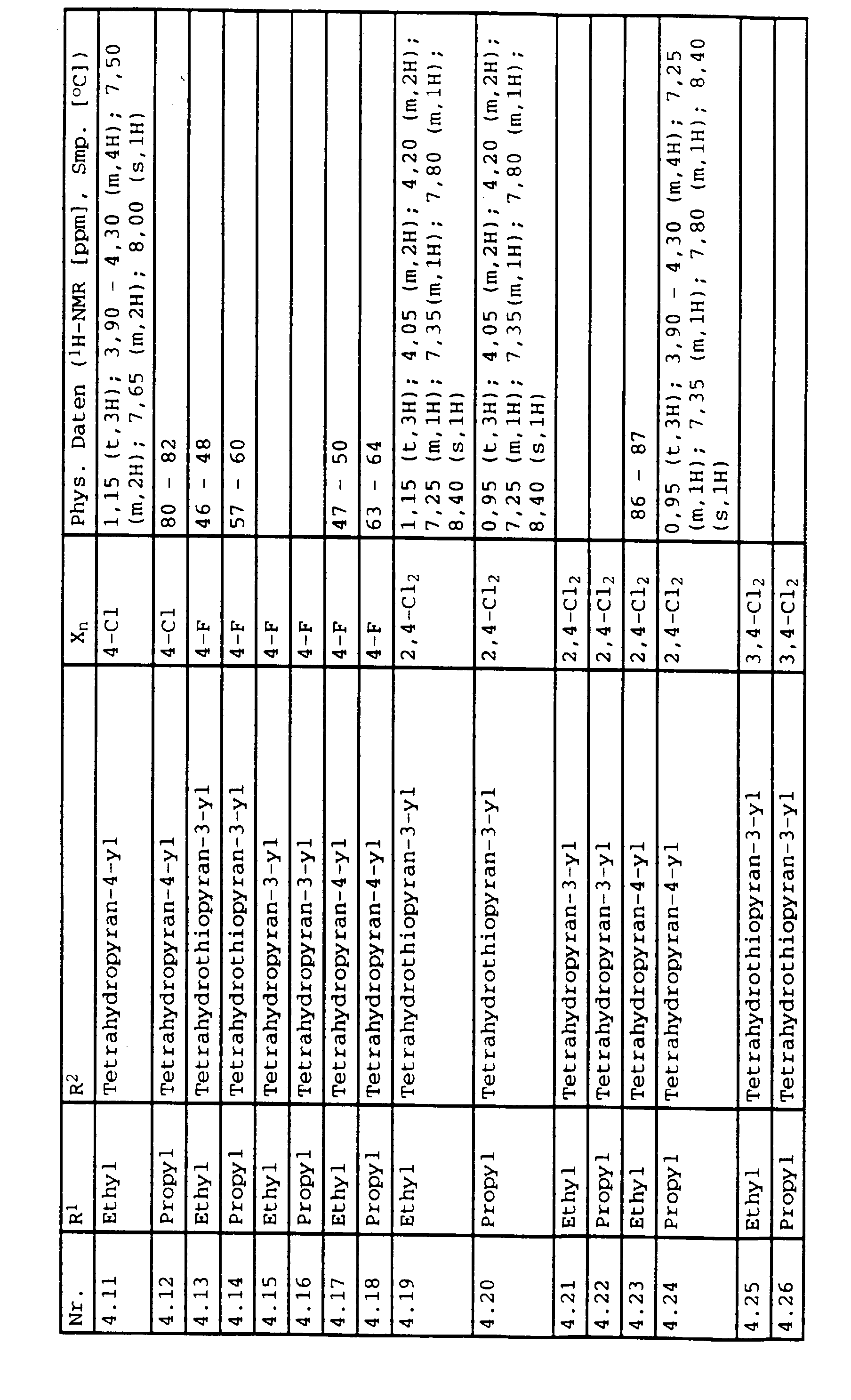

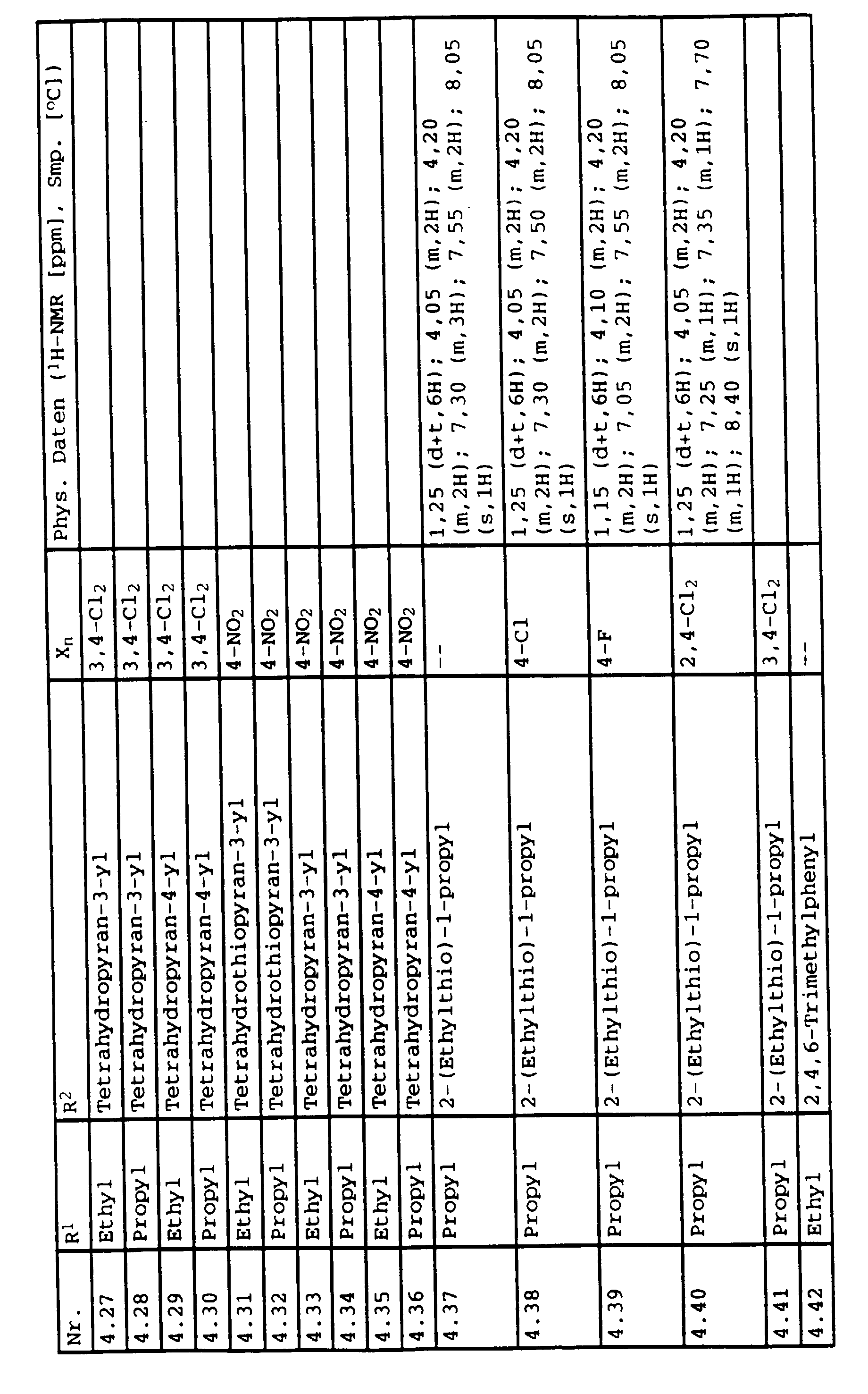

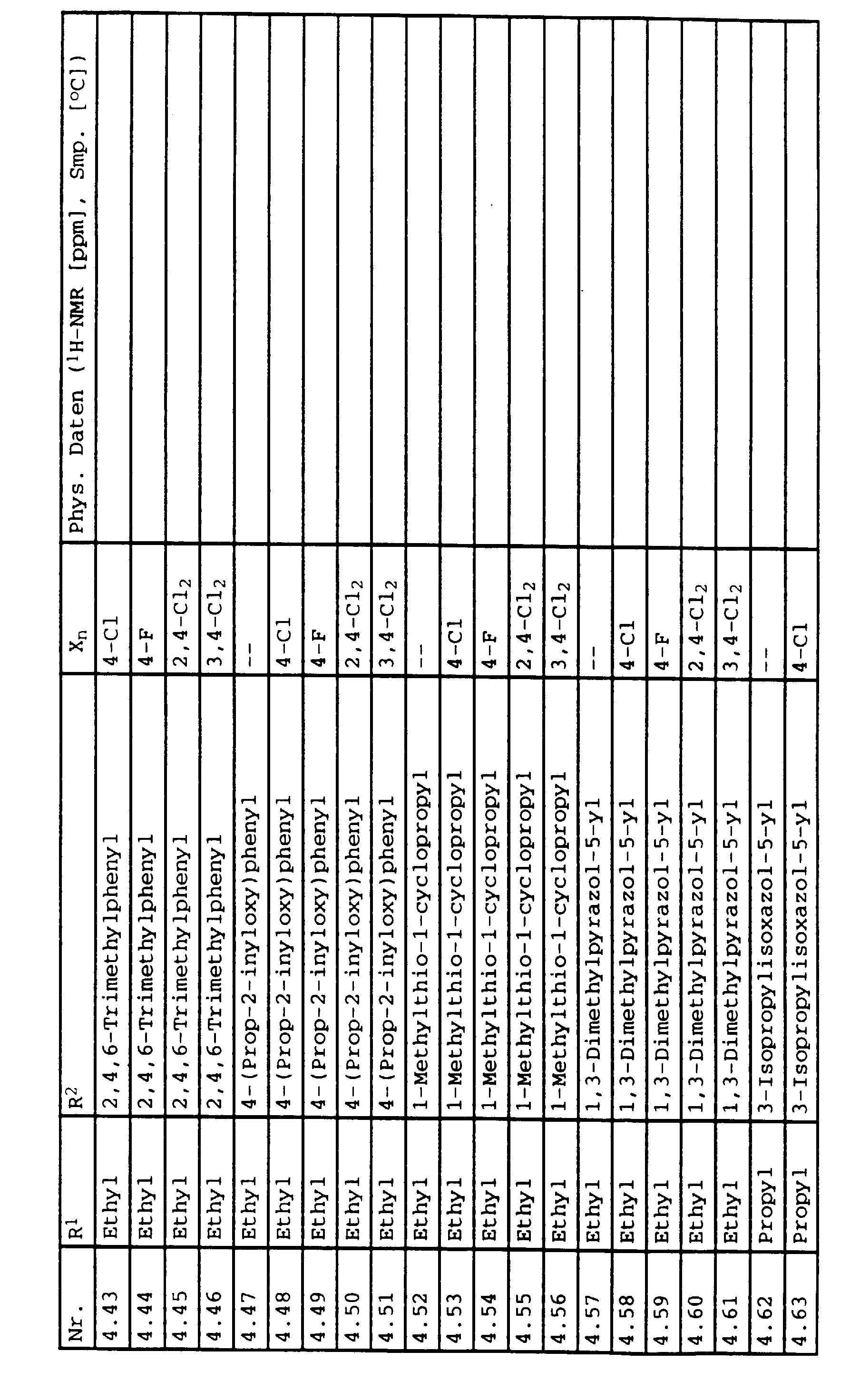

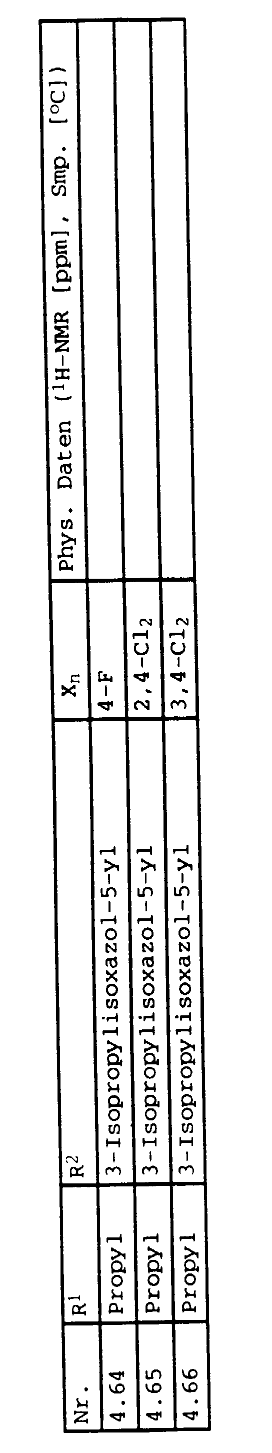

Die Cyclohexenonoximether I eignen sich, sowohl als Isomerengemische als auch in Form der reinen Isomeren, als Herbizide, insbesondere zur Bekämpfung von Pflanzenarten aus der Familie der Gräser (Gramineen). Im allgemeinen sind sie verträglich und somit selektiv in breitblättrigen Kulturen sowie in monokotylen (einkeimblättrigen) Gewächsen, welche nicht zu den Gramineen zählen. Einige der erfindungsgemäßen Cyclohexenonoximether I sind auch zur selektiven Bekämpfung von unerwünschten Gräsern in Gramineenkulturen geeignet.

Die Cyclohexenonoximether I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwekken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergieroder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanole, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt. Beispiele für solche Zubereitungen sind:
I. eine Lösung aus 90 Gew.-Teilen der Verbindung Nr. 2.01 und 10 Gew.-Teilen N-Methyl-α-pyrrolidon, die zur Anwendung in Form kleinster Tropfen geeignet ist;
II. eine Mischung aus 20 Gew.-Teilen der Verbindung Nr. 2.03, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl. Durch feines Verteilen des Gemisches in 100 000 Gew.- Teilen Wasser erhält man eine Dispersion, die 0,02 Gew.-% des Wirkstoffs enthält;
III.eine wäßrige Dispersion aus 20 Gew.-Teilen der Verbindung Nr. 2.37, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes;
IV. eine wäßrige Dispersion aus 20 Gew.-Teilen der Verbindung Nr. 2.43, 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl. Die Mischung dieser Dispersion mit 100 000 Gew.-Teilen Wasser enthält 0,02 % des Wirkstoffes;
V. eine in einer Hammermühle vermahlene Mischung aus 80 Gew.-Teilen der Verbindung Nr. 2.64, 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;
VI. eine innige Mischung aus 3 Gew.-Teilen der Verbindung Nr. 2.70 und 97 Gew.-Teilen feinteiligem Kaolin. Dieses Stäubemittel enthält 3 Gew.-% Wirkstoff;
VII.eine innige Mischung aus 30 Gew.-Teilen der Verbindung Nr. 2.73, 92 Gew.-Teilen pulverförmigem Kieselsäuregel und 8 Gew.-Teilen Paraffinöl, das auf die Oberfläche dieses Kieselsäuregels gesprüht wurde. Diese Aufbereitung gibt dem Wirkstoff eine gute Haftfähigkeit;
VIII. eine stabile wäßrige Dispersion aus 40 Gew.-Teilen der Verbindung Nr. 4.07, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. 4.38, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd Kondensates und 68 Gew.-Teilen eines paraffinischen Mineralöls;
X eine in einer Hammermühle vermahlene Mischung aus 10 Gew.-Teilen der Verbindung Nr. 4.40, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhalt man eine Spritzbrühe, die 0,1 Gew.% des Wirkstoffs enthält.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

In Anbetracht der Vielseitigkeit der Applikationsmethoden können die Cyclohexenonoximether I bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

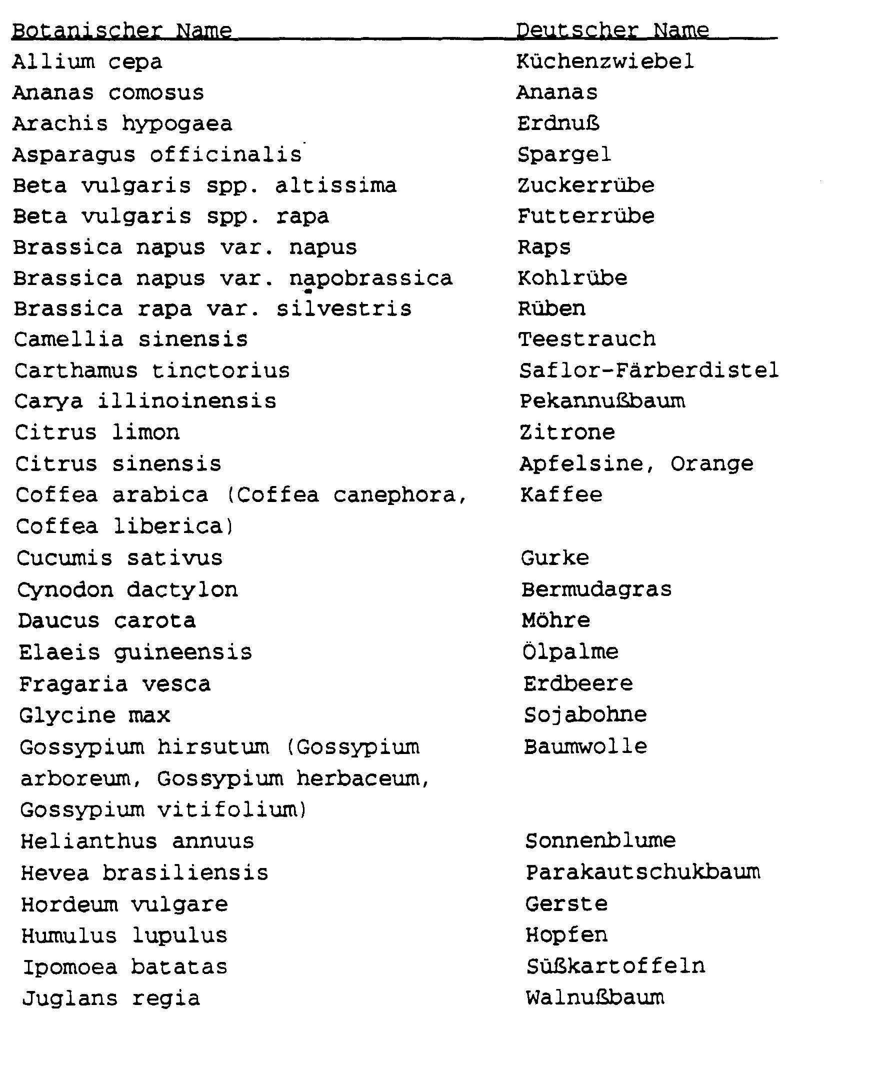

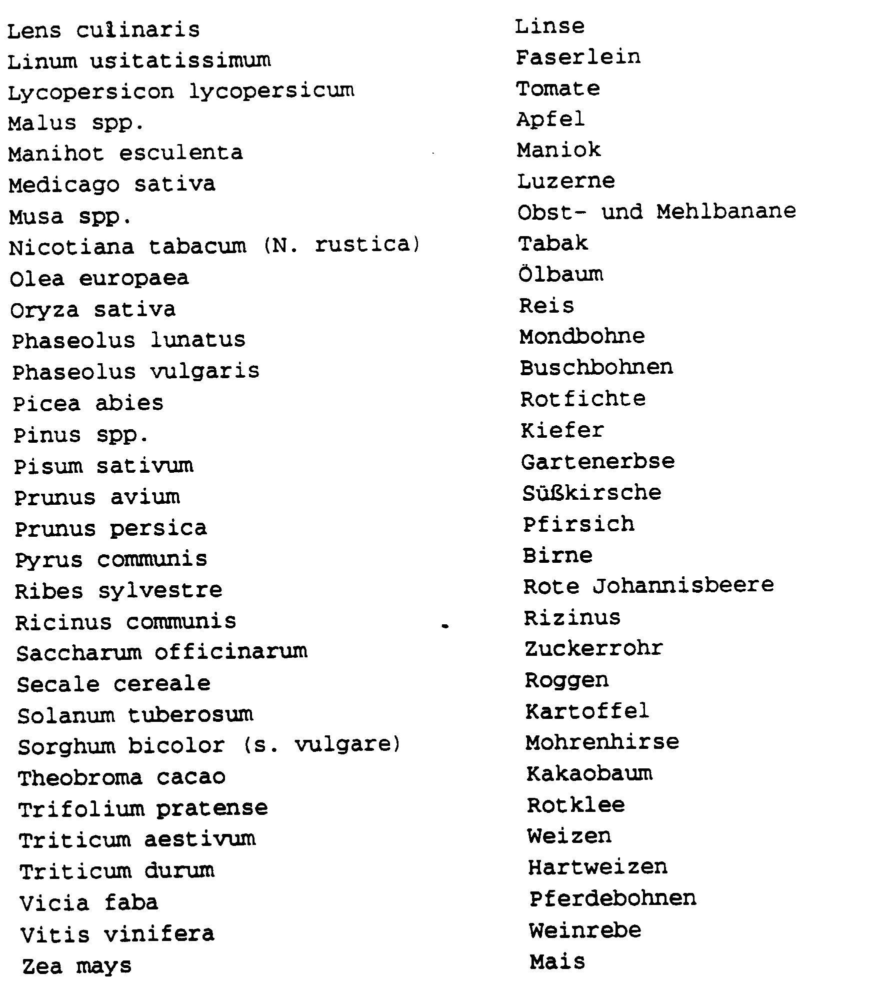

Zur Verbreiterung des Wirkungsspektrums und zur Erzielung synergistischer Effekte können die Cyclohexenonoximether I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsauren, Triazine, Amide, Harnstoffe, Diphenylether, Triazinone, Uracile, Benzofuranderivate, cyclohexan-1,3-dionderivate, die in 2-Stellung z.B. eine Carboxy- oder Carbimino-Gruppe tragen, Chinolincarbonsaurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der ungesättigten Cyclohexenonoximether der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen bereits in den Versuchsgefäßen angezogen oder einige Tage vor der Behandlung in die Versuchsgefäße verpflanzt. Die Applikation der in Wasser suspendierten oder emulgierten Wirkstoffe erfolgte je nach Wuchsform erst bei einer Wuchshöhe von 3 bis 15 cm. Die Aufwandmenge für die Nachauflaufbehandlung betrug 0,03 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 keine Keimung der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Echinochloa crus-galli | Hühnerhirse | barnyardgrass |
| Oryza sativa | Reis | rice |
| Setaria italica | Kolbenhirse | foxtail millet |
| Setaria viridis | Grüne Borstenhirse | green foxtail |

Das Ergebnis zeigte, daß mit Verbindung Nr. 2.13 unerwünschte Gräser in Reis als Beispielkultur sehr gut bekämpft werden können.

## Patentansprüche

1. Cyclohexenonoximether der allgemeinen Formel I 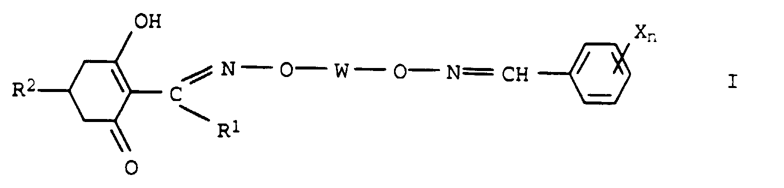 in der die Substituenten und der Index folgende Bedeutung haben:
R¹ eine C₁-C₆-Alkylgruppe;
W eine gewünschtenfalls durch C₁-C₃-Alkyl substituierte C₂-C₄-Alkylenkette;
X Nitro, Cyano, Halogen, C₁-C₄-Alkyl, C₁-C₄-Halogenalkyl;
n 0 bis 3, wobei die Reste X verschieden sein können, wenn n 2 oder 3 bedeutet, oder 1 bis 5 für den Fall, daß alle X Halogen bedeuten;
R² eine C₁-C₄-Alkoxy-C₁-C₆-alkyl- oder C₁-C₄-AlkylthioC₁-C₆-alkylgruppe;
eine C₃-C₇-Cycloalkylgruppe oder eine C₅-C₇-Cycloalkenylgruppe, wobei diese Gruppen gewünschtenfalls ein bis drei Substituenten tragen können, ausgewählt aus einer Gruppe bestehend aus C₁-C₄-Alkyl, C₁-C₄-Alkoxy, C₁-C₄-Alkylthio, C₁-C₄-Halogenalkyl, Hydroxyl und Halogen;
ein 5-gliedriger gesättigter Heterocyclus, der ein oder zwei Sauerstoff- und/oder Schwefelatome als Heteroatome enthält und der gewünschtenfalls noch ein bis drei Substituenten tragen kann, ausgewählt aus einer Gruppe bestehend aus C₁-C₄-Alkyl, C₁-C₄-Alkoxy, C₁-C₄-Alkylthio und C₁-C₄-Halogenalkyl;
ein 6- oder 7-gliedriger Heterocyclus mit einem oder zwei nicht benachtbarten Sauerstoff- und/oder Schwefelatomen als Heteroatomen, der gesättigt oder ein- oder zweifach ungesättigt sein kann, wobei der Heterocyclus gewünschtenfalls noch ein bis drei Substituenten tragen kann, ausgewählt aus einer Gruppe bestehend aus Hydroxyl, Halogen, C₁-C₄-Alkyl, C₁-C₄-Alkoxy, C₁-C₄-Alkylthio und C₁-C₄-Halogenalkyl;
ein 5-gliedriger Heteroaromat, enthaltend ein bis zwei Stickstoffatome und ein Sauerstoff- oder Schwefelatom, wobei dieser Ring gewünschtenfalls noch ein bis drei Substituenten tragen kann, ausgewählt aus einer Gruppe bestehend aus Halogen, Cyano, C₁-C₄-Alkyl, C₁-C₄-Alkoxy, C₁-C₄-Alkylthio, C₁-C₄-Halogenalkyl, C₂-C₆-Alkenyl, C₂-C₆-Alkenyloxy und C₁-C₄-Alkoxy-C₁-C₄-alkyl;
eine Phenyl- oder Pyridylgruppe, wobei diese Gruppen gewünschtenfalls noch ein bis drei der folgenden Substituenten tragen können ausgewählt aus einer Gruppe bestehend aus Halogen, Nitro, Cyano, C₁-C₄-Alkyl, C₁-C₄-Alkoxy, C₁-C₄-Alkylthio, C₁-C₄-Halogenalkyl, C₃-C₆-Alkenyloxy, C₃-C₆-Alkinyloxy und eine Aminogruppe -NR^{a}R^{b}, worin
R^{a} für Wasserstoff, C₁-C₄-Alkyl, C₃-C₆-Alkenyl oder C₃-C₆-Alkinyl steht und
R^{b} für Wasserstoff, C₁-C₄-Alkyl, C₃-C₆-Alkenyl, C₃-C₆-Alkinyl oder C₁-C₆-Acyl steht oder für Benzoyl steht, das gewünschtenfalls seinerseits noch ein bis drei Reste tragen kann, ausgewählt aus einer Gruppe bestehend aus Nitro, Cyano, Halogen, C₁-C₄-Alkyl, C₁-C₄-Alkoxy, C₁-C₄-Alkylthio und C₁-C₄-Halogenalkyl;
sowie die landwirtschaftlich brauchbaren Salze und Ester von C₁-C₁₀-Carbonsäuren und anorganischen Säuren der Verbindungen I.

2. Verfahren zur Herstellung von Cyclohexenonoximethern der Formel I gemäß Anspruch 1, dadurch gekennzeichnet, daß man ein Cyclohexenon der Formel II 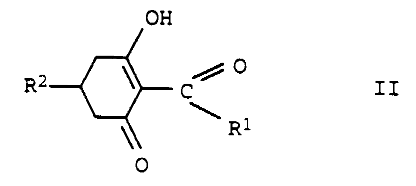 in an sich bekannter Weise in einem inerten organischen Lösungsmittel mit einem Hydroxylamin der Formel III 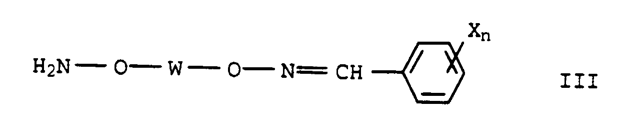 oder mit einem Salz des entsprechenden Hydroxylamins umsetzt.

3. Herbizides Mittel, enthaltend inerte Zusatzstoffe und mindestens einen Cyclohexenonoximether der Formel I gemäß Anspruch 1.

4. Verfahren zur Bekämpfung von unerwünschtem Pflanzenwuchs, dadurch gekennzeichnet, daß man eine herbizid wirksame Menge eines Cyclohexenonoximethers I gemäß Anspruch 1 auf die Pflanzen, deren Lebensraum oder ihr Saatgut einwirken läßt.

5. Hydroxylamine der Formel III 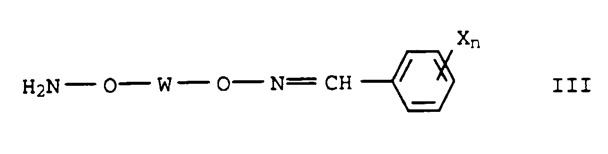 wobei
W für eine gewünschtenfalls durch C₁-C₃-Alkyl substituierte C₂-C₄-Alkylenkette;
X für Nitro, Cyano, Halogen, C₁-C₄-Alkyl, C₁-C₄-Halogenalkyl;
und
n für 0 bis 3, wobei die Reste X verschieden sein können, wenn n 2 oder 3 bedeutet, oder 1 bis 5 für den Fall, daß alle X Halogen bedeuten,
stehen.

## Claims

1. Cyclohexenone oxime ethers of the general formula I 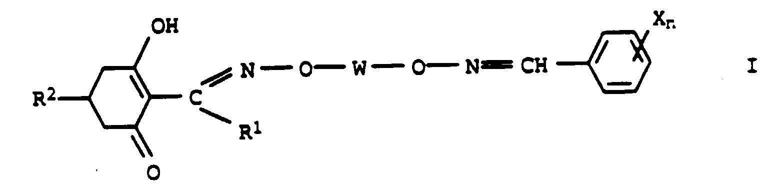 where :
R¹ id C₁-C₆-alkyl;
W is a C₂-C₄-alkylene chain, if desired substituted by C₁-C₃-alkyl;
X is nitro, cyano, halogen, C₁-C₄-alkyl- or C₁-C₄- haloalkyl;
n is 0 to 3, where the radicals X can be different if n is 2 or 3, or 1 to 5 if all the Xs are halogen;
R² is C₁-C₄-alkoxy-C₁-C₆-alkyl or C₁-C₄-alkylthio-C₁-C₆-alkyl;
C₃-C₇-cycloalkyl or C₅-C₇-cycloalkenyl, it being possible if desired for these groups to carry one to three substituents selected from a group consisting of C₁-C₄-alkyl, C₁-C₄-alkoxy, C₁-C₄-alkylthio, C₁-C₄-haloalkyl, hydroxyl and halogen;
a 5-membered saturated heterocycle which contains one or two oxygen and/or sulfur atoms as hetero atoms and which can, if desired, additionally carry one to three substituents selected from a group consisting of C₁-C₄-alkyl, C₁-C₄-alkoxy, C₁-C₄-alkylthio and C₁-C₄-haloalkyl;
a 6- or 7-membered heterocycle having one or two non-adjacent oxygen and/or sulfur atoms as hetero atoms which can be saturated or mono- or diunsaturated, it being possible if desired for the heterocycle additionally to carry one to three substituents selected from a group consisting of hydroxyl, halogen, C₁-C₄-alkyl, C₁-C₄-alkoxy, C₁-C₄-alkylthio and C₁-C₄-haloalkyl;
a 5-membered heteroaromatic, containing one or two nitrogen atoms and an oxygen or sulfur atom, it being possible for this ring if desired additionally to carry one to three substituents selected from a group consisting of halogen, cyano, C₁-C₄-alkyl, C₁-C₄-alkoxy, C₁-C₄-alkylthio, C₁-C₄-haloalkyl, C₂-C₆-alkenyl, C₂-C₆-alkenyloxy and C₁-C₄-alkoxyC₁-C₄-alkyl;
phenyl or pyridyl, it being possible for these groups if desired additionally to carry one to three of the following substituents selected from a group consisting of halogen, nitro, cyano, C₁-C₄-alkyl, C₁-C₄-alkoxy, C₁-C₄-alkylthio, C₁-C₄-haloalkyl, C₃-C₆-alkenyloxy, C₃-C₆-alkynyloxy and an amino group -NR^{a}R^{b}, where
R^{a} is hydrogen, C₁-C₄-alkyl, C₃-C₆-alkenyl or C₃-C₆-alkynyl and
R^{b} is hydrogen, C₁-C₄-alkyl, C₃-C₆-alkenyl, C₃-C₆-alkynyl, C₁-C₆-acyl or benzoyl which if desired can in turn additionally carry one to three radicals selected from a group consisting of nitro, cyano, halogen, C₁-C₄-alkyl, C₁-C₄-alkoxy, C₁-C₄-alkylthio and C₁-C₄-haloalkyl;
and the agriculturally utilizable salts and esters of C₁-C₁₀-carboxylic acids and inorganic acids of the compounds I.

2. A process for preparing cyclohexenone oxime ethers of the formula I as claimed in claim 1, wherein a cyclohexenone of the formula II 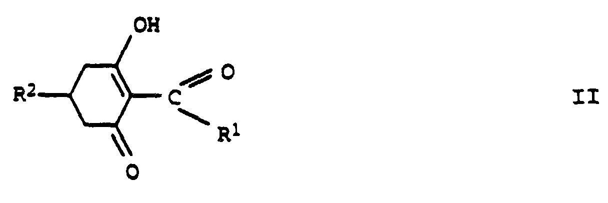 is reacted in a conventional manner in an inert organic solvent with a hydroxylamine of the formula III 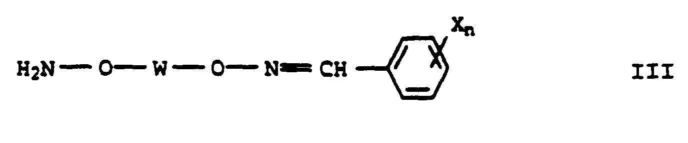 or with a salt of the corresponding hydroxylamine.

3. A herbicidal composition containing inert additives and at least one cyclohexenone oxime ether of the formula I as claimed in claim 1.

4. A method for controlling undesirable plant growth, wherein a herbicidally active amount of cyclohexenone oxime ether I as claimed in claim 1 is allowed to act on the plants, their habitat or their seed.

5. Hydroxylamines of the formula III 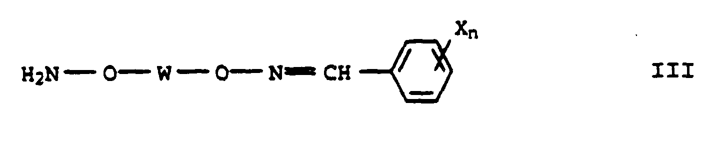 where
W is a C₂-C₄-alkylene chain which, if desired, is substituted by C₁-C₃-alkyl;
X is nitro, cyano, halogen, C₁-C₄-alkyl or C₁-C₄-haloalkyl;
and
n is 0 to 3, where the radicals X can be different if n is 2 or 3, or 1 to 5 if all the Xs are halogen.

## Revendications

1. Ethers de cyclohexénonoximes, de formule générale I 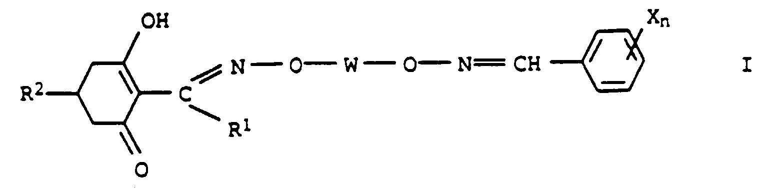 dans laquelle les symboles et les indices ont les significations suivantes :
R¹ représente un groupe alkyle en C1-C6 ;
W représente une chaîne alkylène en C2-C4 éventuellement substituée par un groupe alkyle en C1-C3 ;
X représente un groupe nitro, cyano, un halogène, un groupe alkyle en C1-C4, halogénoalkyle en C1-C4 ;
n est un nombre allant de 0 à 3, les symboles X pouvant avoir les significations différentes lorsque n est égal à 2 ou 3, ou bien un nombre allant de 1 à 5 lorsque tous les symboles X représentent des halogènes ;
R² représente un groupe (alcoxy en C1-C4)alkyle en C1-C6 ou (alkylthio en C1-C4)alkyle en C1-C6 ;
un groupe cycloalkyle en C3-C7 ou un groupe cycloalcényle en C5-C7, ces groupes pouvant éventuellement porter un à trois substituants choisis parmi les groupes alkyle en C1-C4, alcoxy en C1-C4, alkylthio en C1-C4, halogénoalkyle en C1-C4, hydroxy et les halogènes ;
un hétérocycle saturé à cinq chaînons contenant, en tant qu'hétéroatomes, un ou deux atomes d'oxygène et/ou de soufre, et qui peut porter éventuellement un à trois substituants choisis parmi les groupes alkyle en C1-C4, alcoxy en C1-C4, alkylthio en C1-C4 et halogénoalkyle en C1-C4 ;
un hétérocycle à six ou sept chaînons contenant, en tant qu'hétéroatomes, deux atomes d'oxygène et/ou de soufre non voisins, qui est saturé ou peut être mono- ou di-insaturé, l'hétérocycle pouvant encore porter le cas échéant un à trois substituants choisis parmi les groupes hydroxy, les halogènes, les groupes alkyle en C1-C4, alcoxy en C1-C4, alkylthio en C1-C4 et halogénoalkyle en C1-C4 ;
un cycle hétéroaromatique à cinq chaînons contenant un à deux atomes d'azote et un atome d'oxygène ou de soufre, ce cycle pouvant encore porter le cas échéant un à trois substituants choisis parmi les halogènes, les groupes cyano, alkyle en C1-C4, alcoxy en C1-C4, alkylthio en C1-C4, halogénoalkyle en C1-C4, alcényle en C2-C6, alcényle en C2-C6 et (alcoxy en C1-C4)alkyle en C1-C4 ;
un groupe phényle ou pyridyle, ces groupes pouvant encore porter le cas échéant un à trois substituants choisis parmi les halogènes, les groupes nitro, cyano, alkyle en C1-C4, alcoxy en C1-C4, alkylthio en C1-C4, halogénoalkyle en C1-C4, alcényloxy en C3-C6, alcynyloxy en C3-C6 et un groupe amino-NR^{a}R^{b} dans lequel
R^{a} représente l'hydrogène, un groupe alkyle en C1-C4, alcényle en C3-C6 ou alcynyle en C3-C6 et
R^{b} représente l'hydrogène, un groupe alkyle en C1-C4, alcényle en C3C6, alcynyle en C3-C6 ou acyle en C1-C6 ou un groupe benzoyle qui peut lui-même porter un à trois substituants choisis parmi les groupes nitro, cyano, les halogènes, les groupes alkyle en C1-C4, alcoxy en C1-C4, alkylthio en C1-C4 et halogénoalkyle en C1-C4 ;
ainsi que les sels et esters, utilisables dans l'agriculture, des composés I et d'acides carboxyliques en C1-C10 ou d'acides minéraux.

2. Probédé de préparation des éthers de cyclohexénonoximes de formule I de la revendication 1, caractérisé par le fait que l'on fait réagir, de manière connue en soi, une cyclohexénone de formule Il 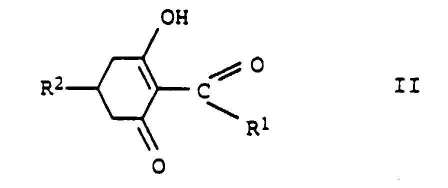 dans un solvant organique inerte, avec une hydroxylamine de formule III 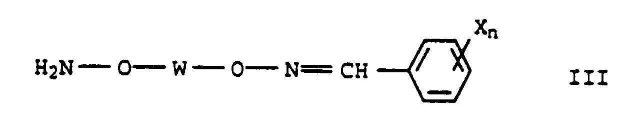 ou avec un sel de cette hydroxylamine.

3. Produit herbicide contenant des additifs inertes et au moins un éther de cyclohexénonoxime de formule I de la revendication 1.

4. Procédé pour combattre les croissances de végétaux indésirables, caractérisé par le fait que l'on fait agir sur les végétaux, leur habitat ou leurs semences, une quantité herbicide efficace d'un éther de cyclohexénonoxime I de la revendication 1.

5. Hydroxylamines de formule III 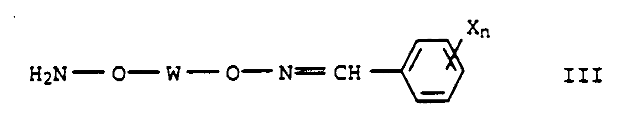 dans laquelle
W représente une chaîne alkylène en C2-C4 éventuellement substituée par un groupe alkyle en C1-C3 ;
X représente un groupe nitro, cyano, un halogène, un groupe alkyle en C1-C4, halogénoalkyle en C1-C4 ; et
n est un nombre allant de 0 à 3, les symboles X pouvant avoir des significations différentes lorsque n est égal à 2 ou 3, ou de 1 à 5 dans le cas où tous les symboles X représentent des halogènes.
